# EUROPÄISCHE PATENTSCHRIFT

(11) **EP 0 830 342 B1**
(45) Veröffentlichungstag und Bekanntmachung des Hinweises auf die Patenterteilung: **09.10.2002**
(21) Anmeldenummer: 96916055.5
(22) Anmeldetag: 13.05.1996
(51) Int. Cl.: C07D 231/22, A01N 43/56, A01N 43/653, C07D 231/18, C07D 249/12, C07D 401/04, C07D 403/04

(54) **AZOLYLOXYBENZYL-ALKOXYACRYLSÄUREESTER, VERFAHREN ZU IHRER HERSTELLUNG UND IHRE VERWENDUNG**
AZOLYL OXYBENZYL ALKOXYACRYLIC ACID ESTERS, PROCESS FOR PRODUCING THEM AND THEIR USE
ESTERS D'ACIDE AZOLYLOXYBENZYL-ALCOXYACRYLIQUE, LEUR PROCEDE DE FABRICATION ET LEUR UTILISATION

(30) Priorität: 24.05.1995 DE 19519041
(43) Veröffentlichungstag der Anmeldung: 25.03.1998
(60) Teilanmeldung: 01107639.5
(73) Patentinhaber: BASF AKTIENGESELLSCHAFT, 67056 Ludwigshafen (DE)
(72) Erfinder: MÜLLER, Bernd, D-67227 Frankenthal (DE); KIRSTGEN, Reinhard, D-67434 Neustadt (DE); KÖNIG, Hartmann, D-69115 Heidelberg (DE); RACK, Michael, D-69123 Heidelberg (DE); OBERDORF, Klaus, D-69117 Heidelberg (DE); RÖHL, Franz, D-67105 Schifferstadt (DE); SAUTER, Hubert, D-68167 Mannheim (DE); LORENZ, Gisela, D-67434 Hambach (DE); AMMERMANN, Eberhard, D-64646 Heppenheim (DE); STRATHMANN, Siegfried, D-67117 Limburgerhof (DE); HARRIES, Volker, D-67227 Frankenthal (DE)
(74) Vertreter: Fitzner, Uwe, Dr.
(86) Internationale Anmeldenummer: EP9602042
(87) Internationale Veröffentlichungsnummer: WO96037477

(56) Entgegenhaltungen:
- EP-A- 0 299 694
- EP-A- 0 581 095
- WO-A-94/00436
- WO-A-95/25095

## Beschreibung

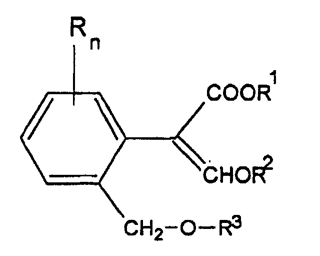

Die vorliegende Erfindung betrifft Verbindungen der allgemeinen Formel I
in der der Index und die Substituenten die folgende Bedeutung haben:
- n: 0, 1, 2, 3 oder 4, wobei die Substituenten R verschieden sein können, wenn n größer als 1 ist;
- R: Nitro, Cyano, Halogen,
C₁-C₁₀-Alkyl, C₂-C₁₀-Alkenyl, C₂-C₁₀-Alkinyl, C₁-C₁₀-Alkoxy, C₂-C₁₀-Alkenyloxy, C₂-C₁₀-Alkinyloxy oder
für den Fall, daß n größer als 1 ist, zusätzlich eine an zwei benachbarte Ringatome gebundene Brücke, welche drei bis vier Glieder aus der Gruppe 3 oder 4 Kohlenstoffatome 1, 2 oder 3 Kohlenstoffatome und 1 oder 2 Stickstoff-, Sauerstoff- und/oder Schwefelatome, wobei diese Brücke gemeinsam mit dem Ring an den sie gebunden ist einen partiell ungesättigten oder aromatischen Rest bilden kann;
wobei die aliphatischen Gruppen partiell oder vollständig halogeniert sein können und/oder einen bis drei, insbesondere einen der folgenden Reste tragen können:
Nitro, Cyano, C₁-C₄-Alkoxy, C₁-C₄-Alkoxycarbonyl oder eine Gruppe = N-OR^{x}, in der R^{x} für C₁-C₆-Alkyl, C₃-C₆-Alkenyl, C₃-C₆-Alkinyl steht,
oder
oder ein ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff-oder Schwefelatom als Ringglieder enthalten kann, welchees wiederum partiell oder vollständig halogeniert sein kann und/oder einen bis drei der folgenden Reste tragen kann: Nitro, Cyano, C₁-C₄-Alkyl, C₁-C₄-Alkoxy, C₁-C₄-Alkoxycarbonyl;
- R¹,R²: C₁-C₄-Alkyl;
- R³: ein Pyrazolrest der Formeln A 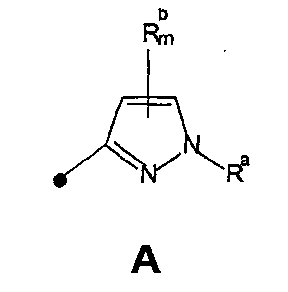 wobei die mit • gekennzeichnete Bindung der Bindung zum Sauerstoff entspricht und in denen der Index und die Substituenten die folgende Bedeutung haben:
- R^{a}: ein partiell oder vollständig halogenierter und/oder durch einen bis drei der folgenden Reste: Nitro, Cyano, C₁-C₄-Alkyl, C₁-C₄-Alkoxy, C₁-C₄-Alkoxycarbonyl, substituierter ein- oder zweikerniger aromatischer Rest, welcher neben Kohlenstoffatomen ein bis vier Stickstoffatome oder ein oder zwei Stickstoffatome und ein oder zwei Sauerstoff-oder Schwefelatome oder ein Sauerstoff- oder ein Schwefelatom als Ringglieder enthalten kann;
- m: 0, 1 oder 2, wobei die Substituenten R^{b} verschieden sein können, wenn m größer als 1 ist;
- R^{b}: Cyano, Nitro, Halogen, C₁-C₄-Alkyl, C₁-C₄-Halogenalkyl, C₁-C₄-Alkoxy, C₁-C₄-Halogenalkoxy, C₁-C₄-Alkylthio und C₁-C₄-Alkoxycarbonyl.

Außerdem betrifft die Erfindung Verfahren zur Herstellung dieser Verbindungen sowie sie enthaltende Mittel und deren Verwendung zur Bekämpfung von tierischen Schädlingen und Schadpilzen.

In der Literatur werden Hetaryloxybenzyl-alkoxyacrylsäureester mit fungizider und z.T. auch insektizider, acarizider und nematodizider Wirkung in allgemeiner Form beschrieben (EP-A 278 595; EP-A 254 426; EP-A 358 692; WO-A 94/19,331, WO-A 94/00,436; EP-A 581 095).Bekannt sind aus der WO 95/25095 auch Verbindungen mit 4-Pyrazolderivaten oder die in der EP-A 299 694 explizit offenbarten Verbindungen mit über CH₂-S-gebundenen Hetarylreste.

Der vorliegenden Erfindung lagen demgegenüber Verbindungen mit verbesserter Wirksamkeit als Aufgabe zugrunde.

Demgemäß wurden die eingangs definierten Verbindungen I gefunden. Außerdem wurden Verfahren zur Herstellung dieser Verbindungen sowie sie enthaltende Mittel und deren Verwendung zur Bekämpfung von tierischen Schädlingen und Schadpilzen gefunden.

Die Verbindungen I sind auf verschiedenen aus der genannten Literatur an sich bekannten Methoden erhältlich.

Beispielsweise erhält man die Verbindungen I durch Umsetzung des Benzylderivats II mit einem Hydroxyazol der Formel III in Gegenwart einer Base.

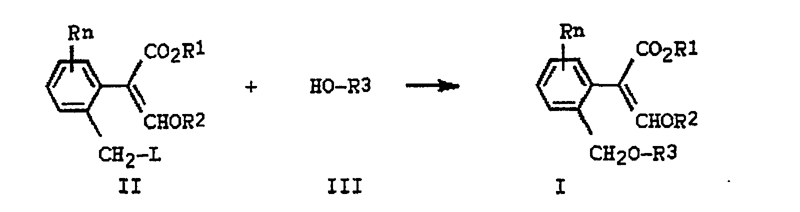

L in der Formel II bedeutet eine nucleophil austauschbare Gruppe, beispielsweise Halogen, z.B. Chlor, Brom und Iod, oder ein Alkyl-oder Arylsulfonat, z.B. Methylsulfonat, Trifluormethylsulfonat, Phenylsulfonat und 4-Methylphenylsulfonat.

Die Umsetzung wird üblicherweise bei Temperaturen von 0°C bis 100°C, vorzugsweise 20°C bis 60°C, durchgeführt.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, i-Propanol, n-Butanol und tert.-Butanol, Ketone wie Aceton und Methylethylketon sowie Essigsäureethylester, Dimethylsulfoxid, Dimethylformamid, N-Methylpyrrolidon, Dimethylacetamid, 1,3-Dimethylimidazolidin-2-on und 1,2-Dimethyltetrahydro-2(1H)-pyrimidin, vorzugsweise Methylenchlorid, Aceton, N-Methylpyrrolidon und Dimethylformamid. Es können auch Gemische der genannten Lösungsmittel verwendet werden.

Als Basen kommen allgemein anorganische Verbindungen wie Alkalimetall- und Erdalkalimetallhydroxide (z.B. Lithiumhydroxid, Natriumhydroxid, Kaliumhydroxid und Calziumhydroxid), Alkalimetall-und Erdalkalimetalloxide (z.B. Lithiumoxid, Natriumoxid, Calziumoxid und Magnesiumoxid), Alkalimetall- und Erdalkalimetallhydride (z.B. Lithiumhydrid, Natriumhydrid, Käliumhydrid und Calziumhydrid), Alkalimetallamide (z.B. Lithiumamid, Natriumamid und Kaliumamid), Alkalimetall- und Erdalkalimetallcarbonate (z.B. Lithiumcarbonat, Kaliumcarbonat und Calziumcarbonat) sowie Alkalimetallhydrogencarbonate (z.B. Natriumhydrogencarbonat), metallorganische Verbindungen, insbesondere Alkalimetallalkyle (z.B. wie Methyllithium, Butyllithium und Phenyllithium), Alkylmagnesiumhalogenide (z.B. Methylmagnesiumchlorid) sowie Alkalimetall- und Erdalkalimetallalkoholate (z.B. Natriummethanolat, Natriumethanolat, Kaliumethanolat, Kalium-tert.-Butanolat und Dimethoxymagnesium), außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Natriummethanolat Kaliumcarbonat, Kaliummethanolat und Kalium-tert.-butanolat.

Die Basen werden im allgemeinen äquimolar, im Überschuß oder gegebenenfalls als Lösungsmittel verwendet.

Es kann für die Umsetzung vorteilhaft sein, eine katalytische Menge eines Kronenethers (z.B. 18-Krone-6 oder 15-Krone-5) zuzusetzen.

Die Umsetzung kann auch in Zweiphasensystemen bestehend aus einer Lösung von Alkali- oder Erdalkalihydroxiden oder -carbonaten in Wasser und einer organischen Phase (z.B. aromatische und/oder halogenierte Kohlenwasserstoffe) durchgeführt werden. Als Phasentransferkatalysatoren kommen hierbei beispielsweise Ammoniumhalogenide und -tetrafluoroborate (z.B. Benzyltriethylammoniumchlorid, Benzyltributylammoniumbromid, Tetrabutylammoniumchlorid, Hexadecyltrimethylammoniumbromid oder Terabutylammoniumtetrafluoroborat) sowie Phosphoniumhalogenide (z.B. Tetrabutylphosphoniumchlorid und Tetraphenylphosphoniumbromid) in Betracht.

Es kann für die Umsetzung vorteilhaft sein, zunächst das Hydroxyazol III mit der Base in das entsprechende Hydroxylat umzusetzen, welchen dann mit dem Benzylderivat umgesetzt wird.

Die für die Herstellung der Verbindungen I benötigten Ausgangsstoffe II sind aus der eingangs zitierten Literatur bekannt oder können nach den dort beschriebenen Verfahren hergestellt werden. Die Ausgangsstoffe III sind aus der Literatur bekannt oder können nach den dort beschriebenen Verfahren hergestellt werden [3-Hydroxypyrazole: J. Heterocycl. Chem. 30, 49 (1993); Chem. Ber. 107, 1318 (1974); Chem. Pharm. Bull. 19, 1389 (1971); Tetrahedron Lett. 11, 875 (1970); Chem. Heterocycl. Comp. 5, 527 (1969); Chem. Ber. 102, 3260 (1969); Chem. Ber. 109, 261 (1976); J. Org. Chem. 31, 1538 (1966); Tetrahedron 43, 607 (1987); 4-Hydroxypyrazole: CA-A 1 177 081; US-A 4,621,144; JP-A 60/155,160; 3-Hydroxytriazole: Chem. Ber. 56, 1794 (1923); DE-A 21 50 169; DE-A 22 00 436; US-A 4,433,148; J. Med. Chem. 33, 2772 (1990); Synthesis 1987, 986; DE-A 22 60 015; DE-A 24 17 970].

Des weiteren erhält man die Verbindungen I durch Umsetzung von a-Ketoestern der Formel IVc im Sinne einer Wittig- oder Wittig-Horner Reaktion {beispielsweise mit (C₆H₅)₃P⁺-CH₂OR² Cl⁻} gemäß der folgenden Reaktionsgleichung (vgl. EP-A 534 216).

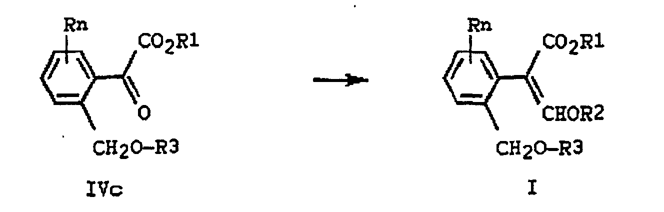

Die Ketoester können analog bekannten Verfahren erhalten werden [vgl. EP-A 493 711; Synth. Commun. 21, 2045 (1991); Synth. Commun. 11, 943 (1981)].

Außerdem können die Verbindungen I dadurch erhalten werden, daß man ein Nitril der Formel IVa mit einem Alkohol (R¹OH) zunächst gemäß EP-A 493 711 in den entsprechenden Benzylester IVb überführt und IVb anschließend gemäß EP-A 203 608 in I überführt.

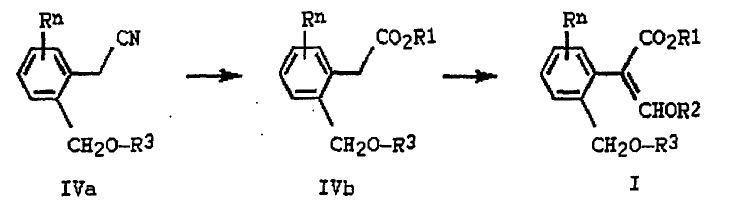

Die Herstellung der Nitrile IVa ist in EP-A 596 692 beschrieben.

Die Verbindungen I können in Bezug auf die Doppelbindung sowohl in der E- als auch in der Z-Konfiguration vorliegen. Beide Isomere können erfindungsgemäß gemeinsam oder getrennt angewendet werden. Bevorzugt ist insbesondere das E-Isomer (Konfiguration bezüglich der Carboxylat-Alkoxy-Gruppe).

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit 1 bis 4 Kohlenstoffatomen, z.B. Methyl, Ethyl, Propyl, 1-Methylethyl, Butyl, 1-Methyl-propyl, 2-Methylpropyl und 1,1-Dimethylethyl;
Halogenalkyl: geradkettige oder verzweigte Alkylgruppen mit 1 bis 4 Kohlenstoffatomen (wie vorstehend genannt), wobei diese in Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkylgruppen mit 1 bis 4 Kohlenstoffatomen (wie vorstehend genannt), welche über ein Sauerstoffatom (-O-) an das Gerüst gebunden sind;
Alkoxycarbonyl: geradkettige oder verzweigte Alkoxygruppen mit 1 bis 4 Kohlenstoffatomen (wie vorstehend genannt), welche über eine Carbonylgruppe (-CO-) an das Gerüst gebunden sind;
Alkylthio: geradkettige oder verzweigte Alkylgruppen mit 1 bis 4 Kohlenstoffatomen (wie vorstehend genannt), welche über ein Schwefelatom (-S-) an das Gerüst gebunden sind;
ggf. subst. Alkyl: gesättigte, geradkettige oder verzweigte Kohlenwasserstoffreste, insbesondere mit 1 bis 10 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylentyl, 1,1-Dimethylbutyl, 1,2-Dimethylbutyl, 1,3-Di-methylbutyl, 2,2-Dimethylbutyl, 2,3-Dimethylbutyl, 3,3-Di-methylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
ggf. subst. Alkenyl: ungesättigte, geradkettige oder verzweigte Kohlenwasserstoffreste, insbesondere mit 2 bis 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Di-methyl-3-butenyl, 1,2-Dimethyl-3-butenyl, 1,3-Dimethyl-1-butenyl, 1,3-Dimethyl-2-butenyl, 1,3-Diethyl-3-butenyl, 2,2-Di-methyl-3-butenyl, 2,3-Dimethyl-1-butenyl, 2,3-Dimethyl-2-butenyl, 2,3-Dimethyl-3-butenyl, 3,3-Dimethyl-l-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-l-methyl-2-propenyl, 1-Ethyl-2-methyl-1-propenyl und 1-Ethyl-2-methyl-2-propenyl;
ggf. subst. Alkinyl: geradkettige oder verzweigte Kohlenwasserstoffgruppen, insbesondere mit 2 bis 20 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl- 1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
ein ggf. subst, gesättigter oder ein- oder zweifach ungesättigter Ring, welcher neben Kohlenstoffatomen ein bis drei der folgenden Heteroatome als Ringglieder enthalten kann: Sauerstoff, Schwefel und Stickstoff, beispielsweise Carbocyclen wie Cyclopropyl, Cyclopentyl, Cyclohexyl, Cyclopent-2-enyl, Cyclohex-2-enyl, 5- bis 6-gliedrige, gesättigte oder ungesättigte Heterocyclen, enthaltend ein bis drei Stickstoffatome und/oder ein Sauerstoff- oder Schwefelatom wie 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl,1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yL, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl und 1,2,4-Tetrahydrotriazin-3-yl, vorzugsweise 2-Tetrahydrofuranyl, 2-Tetrahydrothienyl, 2-Pyrrolidinyl, 3-Isoxazolidinyl, 3-Isothiazolidinyl, 1,3,4-Oxazolidin-2-yl, 2,3-Dihydrothien-2-yl, 4,5-Isoxazolin-3-yl, 3-Piperidinyl, 1,3-Dioxan-5-yl, 4-Piperidinyl, 2-Tetrahydropyranyl, 4-Tetrahydropyranyl;
   oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann, d.h. Arylreste wie Phenyl und Naphthyl, vorzugsweise Phenyl oder 1- oder 2-Naphthyl, und Hetarylreste, beispielsweise 5-Ring Heteroaromaten enthaltend ein bis drei Stickstoffatome und/oder ein Sauerstoff- oder Schwefelatom wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,5-Triazol-3-yl, 1,2,3-Triazol-4-yl, 1,2,3-Triazol-5-yl, 1,2,3-Triazol-4-yl, 5-Tetrazolyl, 1,2,3,4-Thiatriazol-5-yl und 1,2,3,4-Oxatriazol-5-yl, insbesondere 3-Isoxazolyl, 5-Isoxazolyl, 4-Oxazolyl, 4-Thiazolyl, 1,3,4-Oxadiazol-2-yl und 1,3,4-Thiadiazol-2-yl;
Sechsring-Heteroaromaten enthaltend ein bis vier Stickstoffatome als Heteroatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1.2,4-Triazin-3-yl und 1,2,4,5-Tetrazin-3-yl, insbesondere 2-Pyridinyl, 3-Pyridinyl, 4-Pyridinyl, 2-Pyrimidinyl, 4-Pyrimidinyl, 2-Pyrazinyl und 4-Pyridazinyl.

Der Zusatz "ggf. subst" in Bezug auf Alkyl-, Alkenyl- und Alkinylgruppen soll zum Ausdruck bringen, daß diese Gruppen partiell oder vollständig halogeniert sein können (d.h. die Wasserstoffatome dieser Gruppen können teilweise oder vollständig durch gleiche oder verschiedene Halogenatome wie vorstehend genannt (vorzugsweise Fluor, Chlor und Brom, insbesondere Fluor und Chlor) ersetzt sein können und/oder einen bis drei, insbesondere einen, der folgenden Reste tragen können:
Nitro, Cyano, C₁-C₄-Alkoxy, C₁-C₄-Alkoxycarbonyl oder eine Gruppe =N-OR^{x}, in der R^{x} für C₁-C₆-Alkyl, C₃-C₆-Alkenyl und C₃-C₆-Alkinyl steht (R^{x} ist vorzugsweise C₁-C₄-Alkyl),
oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann, d.h. Arylreste wie Phenyl und Naphthyl, vorzugsweise Phenyl oder 1- oder 2-Naphthyl, und Hetarylreste, beispielsweise 5-Ring Heteroaromaten enthaltend ein bis drei Stickstoffatome und/oder ein Sauerstoff- oder Schwefelatom wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,5-Triazol-3-yl, 1,2,3-Triazol-4-yl, 1,2,3-Triazol-5-yl, 1,2,3-Triazol-4-yl, 5-Tetrazolyl, 1,2,3,4-Thiatriazol-5-yl und 1,2,3,4-Oxatriazol-5-yl, insbesondere 3-Isoxazolyl, 5-Isoxazolyl, 4-Oxazolyl, 4-Thiazolyl, 1,3,4-Oxadiazol-2-yl und 1,3,4-Thiadiazol-2-yl;
Sechsring-Heteroaromaten enthaltend ein bis vier Stickstoffatome als Heteroatome wie 2-Pyridinyl, 3-Pyridinyl,' 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl, insbesondere 2-Pyridinyl, 3-Pyridinyl, 4-Pyridinyl, 2-Pyrimidinyl, 4-Pyrimidinyl, 2-Pyrazinyl und 4-Pyridazinyl.

Der Zusatz *"ggf*. *subst"* in Bezug auf die cyclischen (gesättigten, ungesättigtern oder aromatischen) Gruppen soll zum Ausdruck bringen, daß diese Gruppen partiell oder vollständig halogeniert sein können (d.h. die Wasserstoffatome dieser Gruppen können teilweise oder vollständig durch gleiche oder verschiedene Halogenatome wie vorstehend genannt (vorzugsweise Fluor, Chlor und Brom, insbesondere Fluor und Chlor) ersetzt sein können und/oder einen bis drei, der folgenden Reste tragen können: Nitro, Cyano, C₁-C₄-Alkyl, C₁-C₄-Alkoxy und C₁-C₄-Alkoxycarbonyl.

Die bei den Resten genannten ein-oder zweikernigen aromatischen oder heteroaromatischen Systeme können ihrerseits partiell oder vollständig halogeniert sein, d.h. die Wasserstoffatome dieser Gruppen können partiell oder vollständig durch Halogenatome wie Fluor, Chlor, Brom und Jod, vorzugsweise Fluor und Chlor ersetzt sein.

Diese ein- oder zweikernigen aromatischen oder heteroaromatischen Systeme können neben den bezeichneten Halogenatomen zusätzlich ein bis drei der folgenden Substituenten tragen:
Nitro, Cyano, Thiocyanato;
Alkyl, besonders C₁-C₆-Alkyl wie vorstehend genannt, vorzugsweise Methyl, Ethyl, 1-Methylethyl, 1,1-Dimethylethyl, Butyl, Hexyl, insbesondere Methyl und 1-Methylethyl;
C₁-C₄-Halogenalkyl, wie vorstehend genannt, vorzugsweise Trichlormethyl, Difluormethyl, Trifluormethyl, 2,2-Difluorethyl, 2,2,2-Trifluorethyl und Pentafluorethyl;
C₁-C₄-Alkoxy, vorzugsweise Methoxy, Ethoxy, 1-Methylethoxy und 1,1-Dimethylethoxy, insbesondere Methoxy;
C₁-C₄-Halogenalkoxy, besonders C₁-C₂-Halogenalkoxy, vorzugsweise Difluormethyloxy, Trifluormethyloxy und 2,2,2-Trifluorethyloxy, insbesondere Difluormethyloxy;
C₁-C₄-Alkylthio, vorzugsweise Methylthio und 1-Methylethylthio, insbesondere Methylthio;
C₁-C₄-Alkylamino wie Methylamino, Ethylamino, Propylamino, 1-Methylethylamino, Butylamino, 1-Methylpropylamino, 2-Methylpropylamino und 1,1-Dimethylethylamino, vorzugsweise Methylamino und 1,1-Dimethylethylamino, insbesondere Methylamino,
Di-C₁-C₄-alkylamino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)-amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)-amino, N-(1-Methylethyl)-N-(1-methylpropyl)amino, N-(1-Methylethyl)-N-(2-methylpropyl)-amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino, vorzugsweise N,N-Dimethylamino und N,N-Diethylamino, insbesondere N,N-Dimethylamino;
C₁-C₆-Alkylcarbonyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, vorzugsweise Methylcarbonyl, Ethylcarbonyl und 1,1-Dimethylcarbonyl, insbesondere Ethylcarbonyl;
C₁-C₆-Alkoxycarbonyl wie Methoxycarbonyl, Ethoxycarbonyl, Propyloxycarbonyl, 1-Methyl-ethoxycarbonyl, Butyloxycarbonyl, 1-Methylpropyloxycarbonyl, 2-Methylpropyloxycarbonyl, 1,1-Dimethylethoxycarbonyl, Pentyloxycarbonyl, 1-Methylbutyloxycarbonyl, 2-Methylbutyloxycarbonyl, 3-Methylbutyloxycarbonyl, 2,2-Dimethylpropyloxycarbonyl, 1-Ethylpropyloxycarbonyl, Hexyloxycarbonyl, 1,1-Dimethylpropoxycarbonyl, 1,2-Dimethylpropyloxycarbonyl, 1-Methylpentyloxycarbonyl, 2-Methylpentyloxycarbonyl, 3-Methylpentyloxycarbonyl, 4-Methylpentyloxycarbonyl, 1,1-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycarbonyl, 2-Ethylbutyloxycarbonyl, 1,1,2-Trimethylpropyloxycarbonyl, 1,2,2-Trimethylpropyloxycarbonyl, 1-Ethyl-l-methylpropyloxycarbonyl und 1-Ethyl-2-methylpropyloxycarbonyl, vorzugsweise Methoxycarbonyl, Ethoxycarbonyl und 1,1-Dimethylethoxycarbonyl, insbesondere Ethoxycarbonyl;
C₁-C₆-Alkylaminocarbonyl wie Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl, 1,1-Dimethylethylaminocarbonyl, Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2 -Trimethylpropylaminocarbonyl, 1-Ethyl-1-methylpropylaminocarbonyl und 1-Ethyl-2-methylpropylaminöcarbonyl, vorzugsweise Methylamincarbonyl und Ethylamincarbonyl, insbesondere Methylaminocarbonyl;
Di-C₁-C₆-alkylaminocarbonyl, besonders Di-C₁-C₄-alkylaminocarbonyl wie N,N-Dimethylaminocarbonyl, N,N-Diethylaminocarbonyl, N,N-Dipropylaminocarbonyl, N,N-Di-(1-methylethyl)aminocarbonyl, N,N-Dibutylaminocarbonyl, N,N-Di-(1-methylpropyl)aminocarbonyl, N,N-Di-(2-methylpropyl)aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)aminocarbonyl, N-Butyl-N-methylaminocarbonyl, N-Methyl-N-(1-methylpropyl)aminocarbonyl, N-Methyl-N-(2-methylpropyl)aminocarbonyl, N-(1,1-Dimethylethyl)-N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N-(2-methylpropyl)aminocarbonyl, N-Ethyl-N-(1,1-dimethylethyl)-aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)aminocarbonyl, N-(1-Methylethyl)-N-(2-methylpropyl)aminocarbonyl, N-(1,1-Dimethylethyl)-N-(1-methylethyl)aminocarbonyl, N-Butyl-N-(1-methylpropyl)aminocarbonyl, N-Butyl-N-(2-methylpropyl)-aminocarbonyl, N-Butyl-N-(1,1-dimethylethyl)aminocarbonyl, N-(1-Methylpropyl)-N-(2-methyl-propyl)aminocarbonyl, N-(1,1-Dimethylethyl)-N-(1-methylpropyl)aminocarbonyl und N-(1,1-Dimethylethyl)-N-(2-methylpropyl)aminocarbonyl, vorzugsweise N,N-Dimethylaminocarbonyl und N,N-Diethylamincarbonyl, insbesondere N,N-Dimethylaminocarbonyl;
C₁-C₆-Alkylcarboxyl wie Methylcarboxyl, Ethylcarboxyl, Propylcarboxyl, 1-Methylethyl-carboxyl, Butylcarboxyl, 1-Methylpropylcarboxyl, 2-Methylpropylcarboxyl, 1,1-Dimethylethylcarboxyl, Pentylcarboxyl, 1-Methylbutylcarboxyl, 2-Methylbutylcarboxyl, 3-Methylbutylcarboxyl, 1,1-Dimethylpropylcarboxyl, 1,2-Dimethylpropylcarboxyl, 2,2-Dimethylpropylcarboxyl, 1-Ethylpropylcarboxyl, Hexylcarboxyl, 1-Methylpentylcarboxyl, 2-Methylpentylcarboxyl, 3-Methylpentylcarboxyl, 4-Methylpentylcarboxyl, 1,1-Dimethylbutylcarboxyl, 1,2-Dimethylbutylcarboxyl, 1,3-Dimethylbutylcarboxyl, 2,2-Dimethylbutylcarboxyl, 2,3-Dimethylbutylcarboxyl, 3,3-Dimethylbutylcarboxyl, 1-Ethylbutylcarboxyl, 2-Ethylbutylcarboxyl, 1,1,2-Trimethylpropylcarboxyl, 1,2,2-Trimethylpropylcarboxyl, 1-Ethyl-1-methylpropylcarboxyl und 1-. Ethyl-2-methylpropylcarboxyl, vorzugsweise Methylcarboxyl, Ethylcarboxyl und 1,1-Dimethylethylcarbonyl, insbesondere Methylcarboxyl und 1,1-Dimethylethylcarboxyl;
C₁-C₆-Alkylcarbonylamino wie Methylcarbonylamino, Ethylcarbonylamino, Propylcarbonylamino, 1-Methylethylcarbonylamino, Butylcarbonylamino, 1-Methylpropylcarbonylamino, 2-Methylpropylcarbonylamino, 1,1-Dimethylethylcarbonylamino, Pentylcarbonylamino, 1-Methylbutylcarbonylamino, 2-Methylbutylcarbonylamino, 3-Methylbutylcarbonylamino, 2,2-Dimethylpropylcarbonylamino, 1-Ethylpropylcarbonylamino, Hexylcarbonylamino, 1,1-Dimethylpropylcarbonylamino, 1,2-Dimethylpropylcarbonylamino, 1-Methylpentylcarbonylamino, 2-Methylpentylcarbonylamino,3-Methylpentylcarbonylamino, 4-Methylpentylcarbonylamino, 1,1-Dimethylbutylcarbonylamino, 1,2-Dimethylbutylcarbonylamino, 1,3-Dimethylbutylcarbonylamino, 2,2-Dimethylbutylcarbonylamino, 2,3-Dimethylbutylcarbonylamino, 3,3-Dimethylbutylcarbonylamino, 1-Ethylbutylcarbonylamino, 2-Ethylbutylcarbonylamino, 1,1,2-Trimethylpropylcarbonylamino, 1,2,2-Trimethylpropylcarbonylamino, 1-Ethyl-1-methylpropylcarbonylamino und 1-Ethyl-2-methylpropylcarbonylamino, vorzugsweise Methylcarbonylamino und Ethylcarbonylamino, insbesondere Ethylcarbonylamino;
C₁-C₆-Alkylcarbonyl-C₁-C₆-alkylamino wie Methylcarbonyl-methylamino, Ethylcarbonyl-ethylamino, n-Propylcarbonyl-n-propylamino, i-Propylcarbonyl-i-propylamino, Methylcarbonyl-ethylamino, Methylcarbonyl-n-propylamino, Methylcarbonyl-i-propylamino, Ethylcarbonyl-methylamino, Ethylcarbonyl-n-propylamino, Ethylcarbonyl-i-propylamino, n-Propylcarbonyl-methylamino, n-Propylcarbonyl-ethylamino, n-Propylcarbonyl-i-propylamino, i-Propylcarbonyl-methylamino, i-Propylcarbonyl-ethylamino, i-Propylcarbonyln-propylamino, vorzugsweise Methylcarbonyl-methylamino, Methylcarbonyl-ethylamino, Ethylcarbonyl-methylamino, insbesondere Methylcarbonyl-methylamino;
C₃-C₇-Cycloalkyl wie Cyclopropyl, Cyclobutyl, Cyclopentyl, Cyclohexyl und Cycloheptyl, vorzugsweise Cyclopropyl, Cyclopentyl und Cyclohexyl, insbesondere Cyclopropyl;
C₃-C₇-Cycloalkoxy wie Cyclopropyloxy, Cyclobutyloxy, Cyclopentyloxy, Cyclohexyloxy und Cycloheptyloxy, vorzugsweise Cyclopentyloxy und Cyclohexyloxy, insbesondere Cyclohexyloxy;
C₃-C₇-Cycloalkylthio wie Cyclopropylthio, Cyclobutylthio, Cyclopentylthio, Cyclohexylthio und Cycloheptylthio, vorzugsweise Cyclohexylthio;
C₃-C₇-Cycloalkylamino wie Cyclopropylamino, Cyclobutylamino, Cyclopentylamino, Cyclohexylamino und Cycloheptylamino, vorzugsweise Cyclopropylamino und Cyclohexylamino, insbesondere Cyclopropylamino;
weitere Reste für ggf. subst. ein- oder zweikernige aromatische oder heteroaromatische Reste:
Alkenyl, Alkinyl, Halogenalkenyl, Halogenalkinyl, Alkenyloxy, Alkinyloxy, Halogenalkenyloxy, Halogenalkinyloxy, Alkenylthio, Alkinylthio, Alkylsulfoxy, Alkylsulfonyl, Alkenylsulfoxy, Alkinylsulfoxy, Alkinylsulfonyl,
eine Gruppe C(R^{y})=N-OR^{x}, in der R^{x} und R^{y} für ^{c}₁-C₆-Alkyl, C₃-C₆-Alkenyl und C₃-C₅-Alkinyl stehen, R^{x} und R^{y} sind bevorzugt C₁-C₄-Alkyl, Cycloalkenyl, Cycloalkenyloxy, Cycloalkenylthio, Cycloalkenylamino.

Im Hinblick auf ihre biologische Wirksamkeit sind Verbindungen I bevorzugt, in denen R¹ und R² C₁-C₂-Alkyl, insbesondere Methyl, bedeuten.

Des weiteren werden Verbindungen I bevorzugt, in denen n für 0 oder 1, insbesondere 0, steht.

Für den Fall, daß n für 1 steht, werden Verbindungen I bevorzugt, in denen R für eine der folgenden Gruppen steht: Fluor, Chlor, Cyano, Methyl, Trifluormethyl oder Methoxy.

Außerdem werden Verbindungen I, in denen n für 1 steht, bevorzugt, in denen R in der 3- oder 6-Position zum Acrylatrest steht.

Daneben werden Verbindungen I bevorzugt, in denen R^{a} für ggf. subst. C₁-C₄-Alkyl oder C₃-C₆-Cycloalkyl steht.

Gleichermaßen werden Verbindungen I bevorzugt, in denen R^{a} für einen ggf. subst. ein- oder zweikernigen aromatischen oder heteroaromatischen Rest steht.

Des weiteren werden Verbindungen I bevorzugt, in denen R^{a} für einen ggf. subst. 6-Ring-Heteroaromaten, insbesondere Pyridin und Pyrimidin, steht.

Ebenfalls bevorzugt sind Verbindungen I, in denen R^{a} für einen ggf. subst. aromatischen Rest, insbesondere Phenyl, steht.

Insbesondere sind solche Verbindungen I bevorzugt, in denen R^{a} für ggf. substituiertes Phenyl oder Benzyl steht. Als Substituenten des Phenylrestes kommen in diesen Fällen bevorzugt Halogen, Cyano, C₁-C₄-Alkyl, C₁-C₄-Alkoxy, C₁-C₂-Halogenalkyl, C₁-C₂-Halogenalkoxy, C₁-C₄-Alkoxycarbonyl, Phenyl und Oxy-C₁-C₂-alkylidenoxy in Betracht.

Ebenfalls bevorzugt sind Verbindungen I, in denen R^{a} für einen ggf. substituierten Sechsring-Heteroaromaten wie Pyridyl und Pyrimidyl steht. Als Substituenten des Sechsring-Heteroaromaten kommen bevorzugt Cyano, Halogen, C₁-C₄-Alkyl, C₁-C₂-Halogenalkyl, C₁-C₄-Alkoxy, C₁-C₂-Halogenalkoxy und Phenyl in Betracht.

Daneben werden Verbindungen I bevorzugt, in denen m bzw. o für 0 oder 1, insbesondere O, steht.

Gleichermaßen werden Verbindungen I bevorzugt, in denen R^{b} für Cyano, Halogen, C₁-C₄-Alkyl, C₁-C₂-Halogenalkyl, C₁-C₄-Alkoxy, C₁-C₂-Halogenalkoxy und C₁-C₄-Alkoxycarbonyl, insbesondere Fluor, Chlor, Methyl, Trifluormethyl und Methoxycarbonyl, steht.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet (unabhängig von der Kombination, in der sie genannt sind) eine besonders bevorzugte Ausgestaltung des betreffenden Substituenten dar.

### Tabelle 1

Verbindungen der allgemeinen Formel I.1, in denen Rₙ für Wasserstoff steht, R^{b}ₘ für Wasserstoff steht und die Kombination des Index x und der Gruppe R^{x} für eine Verbindung jeweils einer Zeile der Tabelle A entspricht

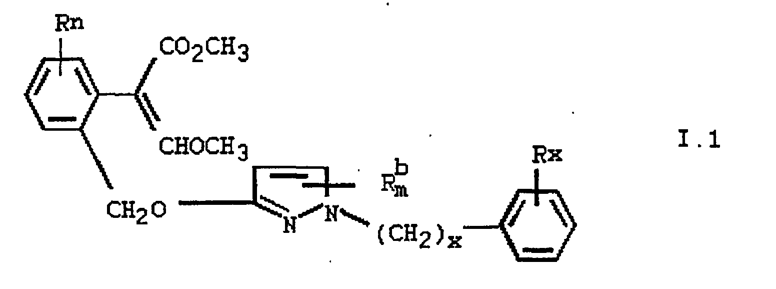

### Tabelle 2

Verbindungen der allgemeinen Formel I.1, in denen Rₙ für Wasserstoff steht, R^{b}ₘ für 5-Methyl steht und die Kombination des Index x und der Gruppe R^{x} für eine Verbindung jeweils einer Zeile der Tabelle A entspricht

### Tabelle 3

Verbindungen der allgemeinen Formel I.1, in denen Rₙ für Wasserstoff steht, R^{b}ₘ für 4-Chlor steht und die Kombination des Index x und der Gruppe R^{x} für eine Verbindung jeweils einer Zeile der Tabelle A entspricht

### Tabelle 4

Verbindungen der allgemeinen Formel I.1, in denen Rₙ für Wasserstoff steht, R^{b}ₘ für 5-CF₃ steht und die Kombination des Index x und der Gruppe R^{x} für eine Verbindung jeweils einer Zeile der Tabelle A entspricht

### Tabelle 5

Verbindungen der allgemeinen Formel I.1, in denen Rₙ für 3-Chlor steht, R^{b}ₘ für Wasserstoff steht und die Kombination des Index x und der Gruppe R^{x} für eine Verbindung jeweils einer Zeile der Tabelle A entspricht

### Tabelle 6

Verbindungen der allgemeinen Formel I.1, in denen Rₙ für 3-Chlor steht, R^{b}ₘ für 5-Methyl steht und die Kombination des Index x und der Gruppe R^{x} für eine Verbindung jeweils einer Zeile der Tabelle A entspricht

### Tabelle 7

Verbindungen der allgemeinen Formel I.1, in denen Rₙ für 3-Chlor steht, R^{b}ₘ für 4-Chlor steht und die Kombination des Index x und der Gruppe R^{x} für eine Verbindung jeweils einer Zeile der Tabelle A entspricht

### Tabelle 8

Verbindungen der allgemeinen Formel I.1, in denen Rₙ für 3-Chlor steht, R^{b}ₘ für 5-CF₃ steht und die Kombination des Index x und der Gruppe R^{x} für eine Verbindung jeweils einer Zeile der Tabelle A entspricht

**Tabelle A**

| **Nr.** | **R**^{**x**} | **X** |
|---|---|---|
| 1 | H | O |
| 2 | 2-F | O |
| 3 | 3-F | O |
| 4 | 4-F | O |
| 5 | 2,4-F₂ | O |
| 6 | 2,4,6-F₃ | O |
| 7 | 2,3,4,5,6-F₅ | O |
| 8 | 2,3-F₂ | O |
| 9 | 2-Cl | O |
| 10 | 3-Cl | O |
| 11 | 4-Cl | O |
| 12 | 2,3-Cl₂ | O |
| 13 | 2,4-Cl₂ | O |
| 14 | 2,5-Cl₂ | O |
| 15 | 2,6-Cl₂ | O |
| 16 | 3,4-Cl₂ | O |
| 17 | 3,5-Cl₂ | O |
| 18 | 2,3,4-Cl₃ | O |
| 19 | 2,3,5-Cl₃ | O |
| 20 | 2,3,6-Cl₃ | O |
| 21 | 2,4,5-Cl₃ | O |
| 22 | 2,4,6-Cl₃ | O |
| 23 | 3,4,5-Cl₃ | O |
| 24 | 2,3,4,6-Cl₄ | O |
| 25 | 2,3,5,6-Cl₄ | O |
| 26 | 2,3,4,5,6-Cl₅ | O |
| 27 | 2-Br | O |
| 28 | 3-Br | O |
| 29 | 4-Br | O |
| 30 | 2,4-Br₂ | O |
| 31 | 2,5-Br₂ | O |
| 32 | 2,6-Br₂ | O |
| 33 | 2,4,6-Br₃ | O |
| 34 | 2,3,4,5,6-Br₅ | O |
| 35 | 2-J | O |
| 36 | 3-J | O |
| 37 | 4-J | O |
| 38 | 2,4-J₂ | O |
| 39 | 2-Cl, 3-F | O |
| 40 | 2-Cl, 4-F | O |
| 41 | 2-Cl 5-F | O |
| 42 | 2-Cl, 6-F | O |
| 43 | 2-Cl, 3-Br | O |
| 44 | 2-Cl, 4-Br | O |
| 45 | 2-Cl, 5-Br | O |
| 46 | 2-Cl, 6-Br | O |
| 47 | 2-Br, 3-Cl | O |
| 48 | 2-Br, 4-Cl | O |
| 49 | 2-Br, 5-Cl | O |
| 50 | 2-Br, 3-F | O |
| 51 | 2-Br, 4-F | O |
| 52 | 2-Br, 5-F | O |
| 53 | 2-Br, 6-F | O |
| 54 | 2-F, 3-Cl | O |
| 55 | 2-F, 4-Cl | O |
| 56 | 2-F, 5-Cl | O |
| 57 | 3-Cl, 4-F | O |
| 58 | 3-Cl, 5-F | O |
| 59 | 3-Cl, 4-Br | O |
| 60 | 3-Cl, 5-Br | O |
| 61 | 3-F, 4-Cl | O |
| 6.2 | 3-F, 4-Br | O |
| 63 | 3-Br, 4-Cl | O |
| 64 | 3-Br, 4-F | O |
| 65 | 2,6-Cl₂, 4-Br | O |
| 66 | 2-CH₃ | O |
| 67 | 3-CH₃ | O |
| 68 | 4-CH₃ | O |
| 69 | 2,3-(CH₃)₂ | O |
| 70 | 2,4-(CH₃)₂ | O |
| 71 | 2,5-(CH₃)₂ | O |
| 72 | 2,6-(CH₃)₂ | O |
| 73 | 3,4-(CH₃)₂ | O |
| 74 | 3,5-(CH₃)₂ | O |
| 75 | 2,3,5-(CH₃)₃ | O |
| 76 | 2,3,4-(CH₃)₃ | O |
| 77 | 2,3,6-(CH₃)₃ | O |
| 78 | 2,4,5-(CH₃)₃ | O |
| 79 | 2,4,6-(CH₃)₃ | O |
| 80 | 3,4,5-(CH₃)₃ | O |
| 81 | 2,3,4,6-(CH₃)₄ | O |
| 82 | 2,3,5,6-(CH₃)₄ | O |
| 83 | 2,3,4.5,6-(CH₃)₅ | O |
| 84 | 2-C₂H₅ | O |
| 85 | 3-C₂H₅ | O |
| 86 | 4-C₂H₅ | O |
| 87 | 2,4-(C₂H₅)₅ | O |
| 88 | 2,6-(C₂H₅)₂ | O |
| 89 | 3,5-(C₂H₅)₂ | O |
| 90 | 2,4,6-(C₂H₅)₃ | O |
| 91 | 2-n-C₃H₇ | O |
| 92 | 3-n-C₃H₇ | O |
| 93 | 4-n-C₃H₇ | O |
| 94 | 2-i-C₃H₇ | O |
| 95 | 3-i-C₃H₇ | O |
| 96 | 4-i-C₃H₇ | O |
| 97 | 2,4-(i-C₃H₇)₂ | O |
| 98 | 2,6-(i-C₃H₇)₂ | O |
| 99 | 3,5-(i-C₃H₇)₂ | O |
| 100 | 2-s-C₄H₉ | O |
| 101 | 3-s-C₄H₉ | O |
| 102 | 4-s-C₄H₉ | O |
| 103 | 2-t-C₄H₉ | O |
| 104 | 3-t-C₄H₉ | O |
| 105 | 4-t-C₄H₉ | O |
| 106 | 4-n-C₉H₁₉ | O |
| 107 | 2-CH₃, 4-t-C₄H₉ | O |
| 108 | 2-CH₃, 6-t-C₄H₉ | O |
| 109 | 2-CH₃, 4-i-C₃H₇ | O |
| 110 | 2-CH₃, 5-i-C₃H₇ | O |
| 111 | 3-CH₃, 4-i-C₃H₇ | O |
| 112 | 2-cyclo-C₆H₁₁ | O |
| 113 | 3-cyclo-C₆H₁₁ | O |
| 114 | 4-cyclo-C₆H₁₁ | O |
| 115 | 2-Cl, 4-C₆H₅ | O |
| 116 | 2-Br, 4-C₆H₅ | O |
| 117 | 2-OCH₃ | O |
| 118 | 3-OCH₃ | O |
| 119 | 4-OCH₃ | O |
| 120 | 2-OC₂H₅ | O |
| 121 | 3-O-C₂H₅ | O |
| 122 | 4-O-C₂H₅ | O |
| 123 | 2-O-n-C₃H₇ | O |
| 124 | 3-O-n-C₃H₇ | O |
| 125 | 4-O-n-C₃H₇ | O |
| 126 | 2-O-i-C₃H₇ | O |
| 127 | 3-O-i-C₃H₇ | O |
| 128 | 4-O-i-C₃H₇ | O |
| 129 | 2-O-n-C₆H₁₃ | O |
| 130 | 3-O-n-C₆H₁₃ | O |
| 131 | 4-O-n-C₆H₁₃ | O |
| 132 | 2-O-CH₂C₆H₅ | O |
| 133 | 3-O-CH₂C₆H₅ | O |
| 134 | 4-O-CH₂C₆H₅ | O |
| 135 | 2-O-(CH₂)₃C₆H₅ | O |
| 136 | 4-O-(CH₂)₃C₆H₅ | O |
| 137 | 2,3-(OCH₃)₂ | O |
| 138 | 2,4-(OCH₃)₂ | O |
| 139 | 2,5-(OCH₃)₂ | O |
| 140 | 2,6-(OCH₃)₂ | O |
| 141 | 3,4-(OCH₃)₂ | O |
| 142 | 3,5-(OCH₃)₂ | O |
| 143 | 2-O-t-C₄H₉ | O |
| 144 | 3-O-t-C₄H₉ | O |
| 145 | 4-O-t-C₄H₉ | O |
| 146 | 3-(3'-Cl-C₆H₄) | O |
| 147 | 4-(4'-CH₃-C₆H₄) | O |
| 148 | 2-O-C₆H₅ | O |
| 149 | 3-O-C₆H₅ | O |
| 150 | 4-O-C₆H₅ | O |
| 151 | 2-O-(2'-F-C₆H₄) | O |
| 152 | 3-O-(3'-Cl-C₆H₄) | O |
| 153 | 4-O-(4'-CH₃-C₆H₄) | O |
| 154 | 2,3,6-(CH₃)₃, 4-F | O |
| 155 | 2,3,6-(CH₃)₃, 4-Cl | O |
| 156 | 2,3,6-(CH₃)₃, 4-Br | O |
| 157 | 2,4-(CH₃)₂, 6-F | O |
| 158 | 2,4-(CH₃)₂, 6-Cl | O |
| 159 | 2,4-(CH₃)₂, 6-Br | O |
| 160 | 2-i-C₃H₇, 4-Cl, 5-CH₃ | O |
| 161 | 2-Cl, 4-NO₂ | O |
| 162 | 2-NO₂, 4-Cl | O |
| 163 | 2-OCH₃, 5-NO₂ | O |
| 164 | 2,4-Cl₂, 5-NO₂ | O |
| 165 | 2,4-Cl₂, 6-NO₂ | O |
| 166 | 2,6-Cl₂, 4-NO₂ | O |
| 167 | 2,6-Br₂, 4-NO₂ | O |
| 168 | 2,6-J₂, 4-NO₂ | O |
| 169 | 2-CH₃, 5-i-C₃H₇, 4-Cl | O |
| 170 | 2-CO₂CH₃ | O |
| 171 | 3-CO₂CH₃ | O |
| 172 | 4-CO₂CH₃ | O |
| 173 | 2-CH₂-OCH₃ | O |
| 174 | 3-CH₂-OCH₃ | O |
| 175 | 4-CH₂-OCH₃ | O |
| 176 | 2-Me-4-CH₃-CH(CH₃)-CO | O |
| 177 | 2-CH₃-4-(CH₃-C=NOCH₃) | O |
| 178 | 2-CH₃-4-(CH₃-C=NOC₂H₅) | O |
| 179 | 2-CH₃-4- (CH₃-C=NO-n-C₃H₇) | O |
| 180 | 2-CH₃-4- (CH₃-C=NO-i-C₃H₇) | O |
| 181 | 2,5-(CH₃)₂-4-(CH₃-C=NOCH₃) | O |
| 182 | 2,5-(CH₃)₂-4-(CH₃-C=NOC₂H₅) | O |
| 183 | 2,5-(CH₃-4-(CH₃-C=NO-n-C₃H₇) | O |
| 184 | 2,5-(CH₃)₂-4-(CH₃-C=NO-i-C₃H₇) | O |
| 185 | 2-C₆H₅ | O |
| 186 | 3-C₆H₅ | O |
| 187 | 4-C₆H₅ | O |
| 188 | 2-(2'-F-C₆H₄) | O |
| 189 | 2-CH₃, 5-Br | O |
| 190 | 2-CH₃, 6-Br | O |
| 191 | 2-Cl, 3-CH₃ | O |
| 192 | 2-Cl, 4-CH₃ | O |
| 193 | 2-Cl, 5-CH₃ | O |
| 194 | 2-F, 3-CH₃ | O |
| 195 | 2-F, 4-CH₃ | O |
| 196 | 2-F, 5-CH₃ | O |
| 197 | 2-Br, 3-CH₃ | O |
| 198 | 2-Br, 4-CH₃ | O |
| 199 | 2-Br, 5-CH₃ | O |
| 200 | 3-CH₃, 4-Cl | O |
| 201 | 3-CH₃, 5-Cl | O |
| 202 | 3-CH₃, 4-F | O |
| 203 | 3-CH₃, 5-F | O |
| 204 | 3-CH₃, 4-Br | O |
| 205 | 3-CH₃, 5-Br | O |
| 206 | 3-F, 4-CH₃ | O |
| 207 | 3-Cl, 4-CH₃ | O |
| 208 | 3-Br, 4-CH₃ | O |
| 209 | 2-Cl, 4,5-(CH₃)₂ | O |
| 210 | 2-Br, 4,5-(CH₃)₂ | O |
| 211 | 2-Cl, 3,5-(CH₃)₂ | O |
| 212 | 2-Br, 3,5-(CH₃)₂ | O |
| 213 | 2,6-Cl₂, 4-CH₃ | O |
| 214 | 2,6-F₂, 4-CH₃ | O |
| 215 | 2,6-Br₂, 4-CH₃ | O |
| 216 | 2,4-Br₂, 6-CH₃ | O |
| 217 | 2,4-F₂, 6-CH₃ | O |
| 218 | 2,4-Br₂, 6-CH₃ | O |
| 219 | 2,6-(CH₃)₂, 4-F | O |
| 220 | 2,6-(CH₃)₂, 4-Cl | O |
| 221 | 2,6-(CH₃)₂, 4-Br | O |
| 222 | 3,5-(CH₃)₂, 4-F | O |
| 223 | 3,5-(CH₃)₂, 4-Cl | O |
| 224 | 3,5-(CH₃)₂, 4-Br | O |
| 225 | 2-CF₃ | O |
| 226 | 3-CF₃ | O |
| 227 | 4-CF₃ | O |
| 228 | 2-OCF₃ | O |
| 229 | 3-OCF₃ | O |
| 230 | 4-OCF₃ | O |
| 231 | 3-OCH₂CHF₂ | O |
| 232 | 2-NO₂ | O |
| 233 | 3-NO₂ | O |
| 234 | 4-NO₂ | O |
| 235 | 2-CN | O |
| 236 | 3-CN | O |
| 237 | 4-CN | O |
| 238 | 2-CH₃, 3-Cl | O |
| 239 | 2-CH₃, 4-Cl | O |
| 240 | 2-CH₃, 5-Cl | O |
| 241 | 2-CH₃, 6-Cl | O |
| 242 | 2-CH₃, 3-F | O |
| 243 | 2-CH₃, 4-F | O |
| 244 | 2-CH₃, 5-F | O |
| 245 | 2-CH₃, 6-F | O |
| 246 | 2-CH₃, 3-Br | O |
| 247 | 2-CH₃, 4-Br | O |
| 248 | 2,5-F₂ | O |
| 249 | 2,6-F₂ | O |
| 250 | 3,4-F₂ | O |
| 251 | 3,5-F₂ | O |
| 252 | H | 1 |
| 253 | 2-F | 1 |
| 254 | 3-F | 1 |
| 255 | 4-F | 1 |
| 256 | 2,4-F₂ | 1 |
| 257 | 2,4,6-F₃ | 1 |
| 258 | 2,3,4,5,6-F₅ | 1 |
| 259 | 2,3-F₂ | 1 |
| 260 | 2-Cl | 1 |
| 261 | 3-Cl | 1 |
| 262 | 4-Cl | 1 |
| 263 | 2,3-Cl₂ | 1 |
| 264 | 2,4-Cl₂ | 1 |
| 265 | 2,5-Cl₂ | 1 |
| 266 | 2,6-Cl₂ | 1 |
| 267 | 3,4-Cl₂ | 1 |
| 268 | 3,5-Cl₂ | 1 |
| 269 | 2,3,4-Cl₃ | 1 |
| 270 | 2,3,5-Cl₃ | 1 |
| 271 | 2,3,6-Cl₃ | 1 |
| 272 | 2,4,5-Cl₃ | 1 |
| 273 | 2,4,6-Cl₃ | 1 |
| 274 | 3,4,5-Cl₃ | 1 |
| 275 | 2,3,4,6-Cl₄ | 1 |
| 276 | 2,3,5,6-Cl₄ | 1 |
| 277 | 2,3,4,5,6-Cl₅ | 1 |
| 278 | 2-Br | 1 |
| 279 | 3-Br | 1 |
| 280 | 4-Br | 1 |
| 281 | 2,4-Br₂ | 1 |
| 282 | 2,5-Br₂ | 1 |
| 283 | 2,6-Br₂ | 1 |
| 284 | 2,4,6-Br₃ | 1 |
| 285 | 2,3,4,5,6-Br₅ | 1 |
| 286 | 2-J | 1 |
| 287 | 3-J | 1 |
| 288 | 4-J | 1 |
| 289 | 2,4-J₂ | 1 |
| 290 | 2-Cl, 3-F | 1 |
| 291 | 2-Cl, 4-F | 1 |
| 292 | 2-Cl, 5-F | 1 |
| 293 | 2-Cl, 6-F | 1 |
| 294 | 2-Cl, 3-Br | 1 |
| 295 | 2-Cl, 4-Br | 1 |
| 296 | 2-Cl, 5-Br | 1 |
| 297 | 2-Cl, 6-Br | 1 |
| 298 | 2-Br, 3-Cl | 1 |
| 299 | 2-Br, 4-Cl | 1 |
| 300 | 2-Br, 5-Cl | 1 |
| 301 | 2-Br, 3-F | 1 |
| 302 | 2-Br, 4-F | 1 |
| 303 | 2-Br, 5-F | 1 |
| 304 | 2-Br, 6-F | 1 |
| 305 | 2-F, 3-Cl | 1 |
| 306 | 2-F, 4-Cl | 1 |
| 307 | 2-F, 5-Cl | 1 |
| 308 | 3-Cl, 4-F | 1 |
| 309 | 3-Cl, 5-F | 1 |
| 310 | 3-Cl, 4-Br | 1 |
| 311 | 3-Cl, 5-Br | 1 |
| 312 | 3-F, 4-Cl | 1 |
| 313 | 3-F, 4-Br | 1 |
| 314 | 3-Br, 4-Cl | 1 |
| 315 | 3-Br, 4-F | 1 |
| 316 | 2,6-Cl₂, 4-Br | 1 |
| 317 | 2-CH₃ | 1 |
| 318 | 3-CH₃ | 1 |
| 319 | 4-CH₃ | 1 |
| 320 | 2,3-(CH₃)₂ | 1 |
| 321 | 2,4-(CH₃)₂ | 1 |
| 322 | 2,5-(CH₃)₂ | 1 |
| 323 | 2,6-(CH₃)₂ | 1 |
| 324 | 3,4-(CH₃)₂ | 1 |
| 325 | 3,5-(CH₃)₂ | 1 |
| 326 | 2,3,5-(CH₃)₃ | 1 |
| 327 | 2,3,4-(CH₃)₃ | 1 |
| 328 | 2,3,6-(CH₃)₃ | 1 |
| 329 | 2,4,5-(CH₃)₃ | 1 |
| 330 | 2,4,6-(CH₃)₃ | 1 |
| 331 | 3,4,5-(CH₃)₃ | 1 |
| 332 | 2,3,4,6-(CH₃)₄ | 1 |
| 333 | 2,3,5,6-(CH₃)₄ | 1 |
| 334 | 2,3,4,5,6-(CH₃)₅ | 1 |
| 335 | 2-C₂H₅ | 1 |
| 336 | 3-C₂H₅ | 1 |
| 337 | 4-C₂H₅ | 1 |
| 338 | 2,4-(C₂H₅)₅ | 1 |
| 339 | 2,6-(C₂H₅)₂ | 1 |
| 340 | 3,5-(C₂H₅)₂ | 1 |
| 341 | 2,4,6-(C₂H₅)₃ | 1 |
| 342 | 2-n-C₃H₇ | 1 |
| 343 | 3-n-C₃H₇ | 1 |
| 344 | 4-n-C₃H₇ | 1 |
| 345 | 2-i-C₃H₇ | 1 |
| 346 | 3-i-C₃H₇ | 1 |
| 347 | 4-i-C₃H₇ | 1 |
| 348 | 2,4-(i-C₃H₇)₂ | 1 |
| 349 | 2.6-(i-C₃H₇)₂ | 1 |
| 350 | 3,5-(i-C₃H₇)₂ | 1 |
| 351 | 2-s-C₄H₉ | 1 |
| 352 | 3-s-C₄H₉ | 1 |
| 353 | 4-s-C₄H₉ | 1 |
| 354 | 2-t-C₄H₉ | 1 |
| 355 | 3-t-C₄H₉ | 1 |
| 356 | 4-t-C₄H₉ | 1 |
| 357 | 4-n-C₉H₁₉ | 1 |
| 358 | 2-CH₃, 4-t-C₄H₉ | 1 |
| 359 | 2-CH₃, 6-t-C₄H₉ | 1 |
| 360 | 2-CH₃, 4-i-C₃H₇ | 1 |
| 361 | 2-CH₃, 5-i-C₃H₇ | 1 |
| 362 | 3-CH₃, 4-i-C₃H₇ | 1 |
| 363 | 2-cyclo-C₆H₁₁ | 1 |
| 364 | 3-cyclo-C₆H₁₁ | 1 |
| 365 | 4-cyclo-C₆H₁₁ | 1 |
| 366 | 2-Cl, 4-C₆H₅ | 1 |
| 367 | 2-Br, 4-C₆H₅ | 1 |
| 368 | 2-OCH₃ | 1 |
| 369 | 3-OCH₃ | 1 |
| 370 | 4-OCH₃ | 1 |
| 371 | 2-OC₂H₅ | 1 |
| 372 | 3-O-C₂H₅ | 1 |
| 373 | 4-O-C₂H₅ | 1 |
| 374 | 2-O-n-C₃H₇ | 1 |
| 375 | 3-O-n-C₃H₇ | 1 |
| 376 | 4-O-n-C₃H₇ | 1 |
| 377 | 2-O-i-C₃H₇ | 1 |
| 378 | 3-O-i-C₃H₇ | 1 |
| 379 | 4-O-i-C₃H₇ | 1 |
| 380 | 2-O-n-C₆H₁₃ | 1 |
| 381 | 3-O-n-C₆H₁₃ | 1 |
| 382 | 4-O-n-C₆H₁₃ | 1 |
| 383 | 2-O-CH₂C₆H₅ | 1 |
| 384 | 3-O-CH₂C₆H₅ | 1 |
| 385 | 4-O-CH₂C₆H₅ | 1 |
| 386 | 2-O-(CH₂)₃C₆H₅ | 1 |
| 387 | 4-O-(CH₂)₃C₆H₅ | 1 |
| 388 | 2,3-(OCH₃)₂ | 1 |
| 389 | 2,4-(OCH₃)₂ | 1 |
| 390 | 2,5-(OCH₃)₂ | 1 |
| 391 | 2,6-(OCH₃)₂ | 1 |
| 392 | 3,4-(OCH₃)₂ | 1 |
| 393 | 3,5-(OCH₃)₂ | 1 |
| 394 | 2-O-t-C₄H₉ | 1 |
| 395 | 3-O-t-C₄H₉ | 1 |
| 396 | 4-O-t-C₄H₉ | 1 |
| 397 | 3-(3'-Cl-C₆H₄) | 1 |
| 398 | 4-(4'-CH₃-C₆H₄) | 1 |
| 399 | 2-O-C₆H₅ | 1 |
| 400 | 3-O-C₆H₅ | 1 |
| 401 | 4-O-C₆H₅ | 1 |
| 402 | 2-O-(2'-F-C₆H₄) | 1 |
| 403 | 3-O-(3'-Cl-C₆H₄) | 1 |
| 404 | 4-O-(4'-CH₃-C₆H₄) | 1 |
| 405 | 2,3,6-(CH₃)₃, 4-F | 1 |
| 406 | 2,3,6-(CH₃)₃, 4-Cl | 1 |
| 407 | 2,3,6-(CH₃)₃, 4-Br | 1 |
| 408 | 2,4-(CH₃)₂, 6-F | 1 |
| 409 | 2,4-(CH₃)₂, 6-Cl | 1 |
| 410 | 2,4-(CH₃)₂, 6-Br | 1 |
| 411 | 2-i-C₃H₇, 4-Cl, 5-CH₃ | 1 |
| 412 | 2-Cl, 4-NO₂ | 1 |
| 413 | 2-NO₂, 4-Cl | 1 |
| 414 | 2-OCH₃, 5-NO₂ | 1 |
| 415 | 2,4-Cl₂, 5-NO₂ | 1 |
| 416 | 2,4-Cl₂, 6-NO₂ | 1 |
| 417 | 2,6-Cl₂, 4-NO₂ | 1 |
| 418 | 2,6-Br₂, 4-NO₂ | 1 |
| 419 | 2,6-J₂, 4-NO₂ | 1 |
| 420 | 2-CH₃, 5-i-C₃H₇, 4-Cl | 1 |
| 421 | 2-CO₂CH₃ | 1 |
| 422 | 3-CO₂CH₃ | 1 |
| 423 | 4-CO₂CH₃ | 1 |
| 424 | 2-CH₂-OCH₃ | 1 |
| 425 | 3-CH₂-OCH₃ | 1 |
| 426 | 4-CH₂-OCH₃ | 1 |
| 427 | 2-Me-4-CH₃-CH(CH₃)-CO | 1 |
| 428 | 2-CH₃-4-(CH₃-C=NOCH₃) | 1 |
| 429 | 2-CH₃-4-(CH₃-C=NOC₂H₅) | 1 |
| 430 | 2-CH₃-4-(CH₃-C=NO-n-C₃H₇) | 1 |
| 431 | 2-CH₃-4-(CH₃-C=NO-i-C₃H₇) | 1 |
| 432 | 2,5-(CH₃)₂-4-(CH₃-C=NOCH₃) | 1 |
| 433 | 2,5-(CH₃)₂-4-(CH₃-C=NOC₂H₅) | 1 |
| 434 | 2,5-(CH₃-4-(CH₃-C=NO-n-C₃H₇) | 1 |
| 435 | 2,5-(CH₃)₂-4-(CH₃-C=NO-i-C₃H₇) | 1 |
| 436 | 2-C₆H₅ | 1 |
| 437 | 3-C₆H₅ | 1 |
| 438 | 4-C₆H₅ | 1 |
| 439 | 2-(2'-F-C₆H₄) | 1 |
| 440 | 2-CH₃, 5-Br | 1 |
| 441 | 2-CH₃, 6-Br | 1 |
| 442 | 2-Cl, 3-CH₃ | 1 |
| 443 | 2-Cl, 4-CH₃ | 1 |
| 444 | 2-Cl, 5-CH₃ | 1 |
| 445 | 2-F, 3-CH₃ | 1 |
| 446 | 2-F, 4-CH₃ | 1 |
| 447 | 2-F, 5-CH₃ | 1 |
| 448 | 2-Br, 3-CH₃ | 1 |
| 449 | 2-Br, 4-CH₃ | 1 |
| 450 | 2-Br, 5-CH₃ | 1 |
| 451 | 3-CH₃, 4-Cl | 1 |
| 452 | 3-CH₃, 5-Cl | 1 |
| 453 | 3-CH₃, 4-F | 1 |
| 454 | 3-CH₃, 5-F | 1 |
| 455 | 3-CH₃, 4-Br | 1 |
| 456 | 3-CH₃, 5-Br | 1 |
| 457 | 3-F, 4-CH₃ | 1 |
| 458 | 3-Cl, 4-CH₃ | 1 |
| 459 | 3-Br, 4-CH₃ | 1 |
| 460 | 2-Cl, 4,5-(CH₃)₂ | 1 |
| 461 | 2-Br, 4,5-(CH₃)₂ | 1 |
| 462 | 2-Cl, 3,5-(CH₃)₂ | 1 |
| 463 | 2-Br, 3,5-(CH₃)₂ | 1 |
| 464 | 2,6-Cl₂, 4-CH₃ | 1 |
| 465 | 2,6-F₂, 4-CH₃ | 1 |
| 466 | 2,6-Br₂, 4-CH₃ | 1 |
| 467 | 2,4-Br₂, 6-CH₃ | 1 |
| 468 | 2,4-F₂, 6-CH₃ | 1 |
| 469 | 2,4-Br₂, 6-CH₃ | 1 |
| 470 | 2,6-(CH₃)₂, 4-F | 1 |
| 471 | 2,6-(CH₃)₂, 4-Cl | 1 |
| 472 | 2,6-(CH₃)₂, 4-Br | 1 |
| 473 | 3,5-(CH₃)₂, 4-F | 1 |
| 474 | 3,5-(CH₃)₂, 4-Cl | 1 |
| 475 | 3,5-(CH₃)₂, 4-Br | 1 |
| 476 | 2-CF₃ | 1 |
| 477 | 3-CF₃ | 1 |
| 478 | 4-CF₃ | 1 |
| 479 | 2-OCF₃ | 1 |
| 480 | 3-OCF₃ | 1 |
| 481 | 4-OCF₃ | 1 |
| 482 | 3-OCH₂CHF₂ | 1 |
| 483 | 2-NO₂ | 1 |
| 484 | 3-NO₂ | 1 |
| 485 | 4-NO₂ | 1 |
| 486 | 2-CN | 1 |
| 487 | 3-CN | 1 |
| 488 | 4-CN | 1 |
| 489 | 2-CH₃, 3-Cl | 1 |
| 490 | 2-CH₃, 4-Cl | 1 |
| 491 | 2-CH₃, 5-Cl | 1 |
| 492 | 2-CH₃, 6-Cl | 1 |
| 493 | 2-CH₃, 3-F | 1 |
| 494 | 2-CH₃, 4-F | 1 |
| 495 | 2-CH₃, 5-F | 1 |
| 496 | 2-CH₃, 6-F | 1 |
| 497 | 2-CH₃, 3-Br | 1 |
| 498 | 2-CH₃, 4-Br | 1 |
| 499 | 2,5-F₂ | 1 |
| 500 | 2,6-F₂ | 1 |
| 501 | 3,4-F₂ | 1 |
| 502 | 3,5-F₂ | 1 |

Die erfindungsgemäßen Verbindungen der Formel I eignen sich zur Bekämpfung von Schadpilzen und von tierischen Schädlingen aus der Klasse der Insekten, Spinnentiere und Nematoden. Sie können im Pflanzenschutz sowie auf dem Hygiene-, Vorratsschutz- und Veterinärsektor als Fungizide und Schädlingsbekämpfungsmittel eingesetzt werden.

Zu den schädlichen Insekten gehören:
aus der Ordnung der Schmetterlinge (Lepidoptera) beispielsweise Adoxophyes orana, Agrotis ypsilon, Agrotis segetum, Alabama argillacea, Anticarsia gemmatalis, Argyresthia conjugella, Autographa gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha, Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis, Thaumatopoea pityocampa, Tortrix viridana, Trichoplusia ni, Tryporyza incertulas, Zeiraphera canadensis,
ferner Galleria mellonella und Sitotroga cerealella, Ephestia cautella, Tineola bisselliella;
aus der Ordnung der Käfer (Coleoptera) beispielsweise Agriotes lineatus, Agriotes obscurus, Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllopertha horticola, Phyllophaga sp., Phyllotreta chrysocephala, Phyllotreta nemorum, Phyllotreta striolata, Popillia japonica, Psylliodes napi, Scolytus intricatus, Sitona lineatus,
ferner Bruchus rufimanus, Bruchus pisorum, Bruchus lentis, Sitophilus granaria, Lasioderma serricorne, Oryzaephilus surinamensis, Rhyzopertha dominica, Sitophilus oryzae, Tribolium castaneum, Trogoderma granarium, Zabrotes subfasciatus;
aus der Ordnung der Zweiflügler (Diptera) beispielsweise Anastrepha ludens, Ceratitis capitata, Contarinia sorghicola, Dacus cucurbitae, Dacus oleae, Dasineura brassicae, Delia coarctata, Delia radicum, Hydrellia griseola, Hylemyia platura, Liriomyza sativae, Liriomyza trifolii, Mayetiola destructor, Orseolia oryzae, Oscinella frit, Pegomya hyoscyami, Phorbia antiqua, Phorbia brassicae, Phorbia coarctata, Rhagoletis cerasi, Rhagoletis pomonella, Tipula oleracea, Tipula paludosa,
ferner Aedes aegypti, Aedes vexans, Anopheles maculipennis, Chrysomya bezziana, Chrysomya hominivorax, Chrysomya macellaria, Cordylobia anthropophaga, Culex pipiens, Fannia canicularis, Gasterophilus intestinalis, GLossina morsitans, Haematobia irritans, Haplodiplosis equestris, Hypoderma lineata, Lucilia caprina, Lucilia cuprina, Lucilia sericata, Musca domestica, Muscina stabulans, Oestrus ovis, Tabanus bovinus, Simulium damnosum;
aus der Ordnung der Thripse (Thysanoptera) beispielsweise Frankliniella fusca, Frankliniella occidentalis, Frankliniella tritici, Haplothrips tritici, Scirtothrips citri, Thrips oryzae, Thrips palmi, Thrips tabaci;
aus der Ordnung der Hautflügler (Hymenoptera) beispielsweise Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri;
aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus hesperus, Lygus lineolaris, Lygus pratensis, Nezara viridula, Piesma quadrata, Solubea insularis, Thyanta perditor;
aus der Ordnung der Pflanzensauger (Homoptera) beispielsweise Acyrthosiphon onobrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Planococcus citri, Psylla mali, Psylla piri, Psylla pyricol, Quadraspidiotus perniciosus, Rhopalosiphum maidis, Saissetia oleae, Schizaphis graminum, Selenaspidus articulatus, Sitobion avenae, Sogatella furcifera, Toxoptera citricida, Trialeurodes abutilonea, Trialeurodes vaporariorum, Viteus vitifolii;
aus der Ordnung der Termiten (Isoptera) beispielsweise Calotermes flavicollis, Leucotermes flavipes, Macrotermes subhyalinus, Odontotermes formosanus, Reticulitermes lucifugus, Termes natalensis;
aus der Ordnung der Geradflügler (Orthoptera) beispielsweise Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria,
ferner Acheta domestica, Blatta orientalis, Blattella germanica, Periplaneta americana;
aus der Ordnung der Arachnoidea beispielsweise phytophage Milben wie Aculops lycopersicae, Aculops pelekassi, Aculus schlechtendali, Brevipalpus phoenicis, Bryobia praetiosa, Eotetranychus carpini, Eutetranychus banksii, Eriophyes sheldoni, Oligonychus pratensis; Panonychus ulmi, Panonychus citri, Phyllocoptruta oleivora, Polyphagotarsonemus latus, Tarsonemus pallidus, Tetranychus cinnabarinus, Tetranychus kanzawai, Tetranchus pacificus, Tetranychus urticae, Zecken wie Amblyomma americanum, Amblyomma variegatum,. Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Rhipicephalus appendiculatus und Rhipicephalus evertsi sowie tierparasitische Milben wie Dermanyssus gallinae, Psoroptes ovis und Sarcoptes scabiei;
aus der Klasse der Nematoden beispielsweise Wurzelgallennematoden, z.B. Meloidogyne hapla, Meloidogyne incognita, Meloidogyne javanica, zystenbildende Nematoden, z.B. Globodera pallida, Globodera rostochiensis, Heterodera avenae, Heterodera glycines, Heterodera schachtii, migratorische Endoparasiten und semi-endoparasitische Nematoden, z.B. Heliocotylenchus multicinctus, Hirschmanniella oryzae, Hoplolaimus spp, Pratylenchus brachyurus, Pratylenchus fallax, Pratylenchus penetrans, Pratylenchus vulnus, Radopholus similis, Rotylenchus reniformis, Scutellonema bradys, Tylenchulus semipenetrans, Stock- und Blattnematoden z.B. Anguina tritici, Aphelenchoides besseyi, Ditylenchus angustus, Ditylenchus dipsaci, Virusvektoren, z.B. Longidorus spp, Trichodorus christei, Trichodorus viruliferus, Xiphinema index, Xiphinema mediterraneum.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Als Fungizide sind die a-Phenylbutensäuremethylester der Formel I z.T. systemisch wirksam. Sie können als Blatt- und Bodenfungizide gegen ein breites Spektrum von pflanzenpathogenen Pilzen, insbesondere aus der Klasse der Ascomyceten, Deuteromyceten, Phycomyceten und Basidiomyceten eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zukkerrohr, Wein, Obst- und Zierpflanzen und Gemüsepflanzen wie Gurken, Bohnen und Kürbisgewächsen, sowie an den Samen dieser Pflanzen.

Speziell eignen sich die Verbindungen I zur Bekämpfung folgender Pflanzenkrankheiten:
* Erysiphe graminis (echter Mehltau) in Getreide,
* Erysiphe cichoracearum und Sphaerotheca fuliginea an Kürbisgewächsen,
* Podosphaera leucotricha an Äpfeln,
* Uncinula necator an Reben,
* Puccinia-Arten an Getreide,
* Rhizoctonia-Arten an Baumwolle und Rasen,
* Ustilago-Arten an Getreide und Zuckerrohr,
* Venturia inaequalis (Schorf) an Äpfeln,
* Helminthosporium-Arten an Getreide,
* Septoria nodorum an Weizen,
* Botrytis cinerea (Grauschimmel) an Erdbeeren, Reben,
* Cercospora arachidicola an Erdnüssen,
* Pseudocercosporella herpotrichoides an Weizen, Gerste,
* Pyricularia oryzae an Reis,
* Phytophthora infestans an Kartoffeln und Tomaten,
* Fusarium- und Verticillium-Arten an verschiedenen Pflanzen,
* Plasmopara viticola an Reben,
* Alternaria-Arten an Gemüse und Obst.

Die neuen Verbindungen können auch im Materialschutz (Holzschutz) eingesetzt werden, z.B. gegen Paecilomyces variotii.

Sie können in die üblichen Formulierungen übergeführt werden, wie Lösungen, Emulsionen, Suspensionen, Stäube, Pulver, Pasten oder Granulate. Die Anwendungformen richten sich dabei nach dem jeweiligen Verwendungszweck; sie sollten in jedem Fall möglichst die feinste Verteilung der Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht:
- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;
- Trägerstoffe wie natürliche Gesteinsmehle (z.B. Kaoline, Ton-erden, Talkum, Kreide) und synthetische Gesteinsmehle (z.B. hochdisperse Kieselsäure, Silikate);
- Emulgiermittel wie nichtionogene und anionische Emulgatoren (z.B. Polyoxyethylen-Fettalkohol-Ether, Alkylsulfonate und Arylsulfonate) und
- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Wirkstoffkonzentrationen in den anwendungsfertigen Zubereitungen können in größeren Bereichen variiert werden.

Ganz allgemein enthalten die Mittel zwischen 0,0001 und 95 Gew.-% Wirkstoff.

Formulierungen mit mehr als 95 Gew.-% Wirkstoff können mit gutem Erfolg im Ultra-Low-VolumeVerfahren (ULV) ausgebracht werden, wobei sogar der Wirksoff ohne Zusätze verwendet werden kann.

Für die Anwendung als Fungizide empfehlen sich Konzentrationen zwischen 0,01 und 95 Gew.%, vorzugsweise zwischen 0,5 und 90 Gew.%, Wirkstoff. Für die Anwendung als Insektizide kommen Formulierungen mit 0,0001 bis 10 Gew.%, vorzugsweise 0,01 bis 1 Gew.% Wirkstoff, in Betracht.

Die Wirkstoffe werden normalerweise in einer Reinheit von 90 % bis 100 %, vorzugsweise 95 % bis 100 % (nach NMR-Spektrum) eingesetzt.

Beispiele für solche Zubereitungen sind:
I. eine Lösung aus 90 Gew.-Teilen einer erfindungsgemäßen Verbindung I und 10 Gew.-Teilen N-Methyl-a-pyrrolidon, die zur Anwendung in Form kleinster Tropfen geeignet ist;
II. eine Lösung von 20 Gew.-Teilen einer erfindungsgemäßen Verbindung I in einer Mischung aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylen oxid an 1 Mol Ricinusöl; durch feines Verteilen der Formulierung in Wasser erhält man eine Dispersion.
III. eine Lösung von 20 Gew.-Teilen einer erfindungsgemäßen Verbindung I in einer Mischung aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl; durch feines Verteilen der Formulierung in Wasser erhält man eine Dispersion.
IV. eine wäßrige Dispersion aus 20 Gew.-Teilen einer erfindungsgemäßen Verbindung I, in einer Mischung aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl; durch feines Verteilen der Formulierung in Wasser erhält man eine Dispersion.
V. eine in einer Hammermühle vermahlene Mischung aus 20 Gew.-Teilen einer erfindungsgemäßen Verbindung I, 3 Gew.-Teilen des Natriumsalzes der Diisobutylnaphtalin-asulfonsäure, 17 Gew.-Teilen des Natriumsalzes einer Ligninsulfonsäure aus einer Sulfitablauge und 60 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 92 Gew.-Teilen pulverförmigem Kieselsäuregel und 8 Gew.Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenosulfonsäure-harnstoffformaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkohol-polyglykol-ether, 2 Gew.-Teilen des Natriumsalzes eines Phenolsulfonsäure-harnstoff-formaldehyd-Kondensates und 68 Gew.-Teilen eines paraffinischen Mineralöls;
X. eine in einer Hammermühle vermahlene Mischung aus 10 Gew.-Teilen einer erfindungsgemäßen Verbindung I, 4 Gew.-Teilen des Natriumsalzes der Diisobutylnaphthalina-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Verbindungen I werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die Aufwandmengen liegen je nach Art des gewünschten Effektes zwischen 0,02 und 3 kg Wirkstoff pro ha, vorzugsweise bei 0,1 bis 1 kg/ha.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Schädlingen beträgt unter Freilandbedingungen 0,02 bis 10, vorzugsweise 0,1 bis 2,0 kg/ha Wirkstoff.

Die Verbindungen I, allein oder in Kombination mit Herbiziden oder Fungiziden, können auch noch mit weiteren Pflanzenschutzmitteln gemischt gemeinsam ausgebracht werden, beispielsweise mit Wachstumsregulatoren oder mit Mitteln zur Bekämpfung von Schädlingen oder Bakterien. Von Interesse ist ferner die Mischbarkeit mit Düngemitteln oder mit Mineralsalzlösungen, welche zur Behebung von Ernährungs- und Spurenelementmängeln eingesetzt werden.

Die Pflanzenschutz- und Düngemittel können zu den erfindungsgemäßen Mitteln im Gewichtsverhältnis 1:10 bis 10:1 zugesetzt werden, gegebenenfalls auch erst unmittelbar vor der Anwendung (Tankmix). Beim Vermischen mit Fungiziden oder Insektiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid; Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthals°ure-di-isopropylester;
heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-b-[bis-(dimethylamino)-phosphinyl]-3-phenyl-1,2,4-triazol, 2,3-Dicyano-1,4-dithioanthrachinon, 2-Thio-1,3-dithiolo-b-[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid, N-Dichlorfluormethylthio-N',N'dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethylfuran-3-carbonsäurecyclohexylamid, N-Cyclohexyl-N-methoxy-2,5-dimethylfuran-3-carbonsäureamid, 2-Methyl-benzoesäure-anilid, 2-Iodbenzoesäure-anilid, N-Formyl-N-morpholin-2,2,2-trichlorethylacetal, Piperazin-1,4-diylbis-(1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclodedecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-ylethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, a-(2-Chlorphenyl)-a-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-a-(1H-1,2,4-triazolyl-1-methyl)-benzhydrylalkohol, N-(3-Chlor-2,6-dinitro-4-trifluormethylphenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Daten aufgeführt.
a-(3-Chlor-2-((1-(2,4-dichlorphenyl) -propyl-3)-oxymethyl)-phenyl)-b-methoxy-acrylsäuremethylester 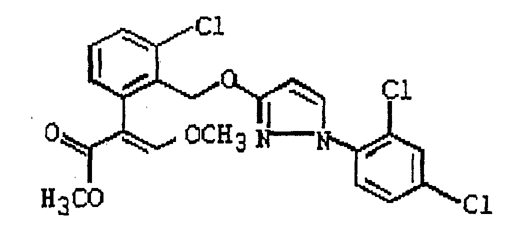
a) (3-Chlor-2-methylphenyl)-glyoxalsäuremethylester
   30 g 2,6-Dichlortoluol wurden mit 24 g Mg-Spänen in 0,6 l THF zum 3-Chlor-2-methylphenyl-magnesiumchlorid umgesetzt. Man legte eine auf -20 bis -25°C gekühlte Lösung von 152 g Oxalylchlorid in 1 l THF vor und tropfte hierzu die obige Grignard-Lösung. Nach ca. 30 min ließ man noch eine weitere Stunde bei -50°C rühren und tropfte dann innerhalb von 2 h 256 g Methanol hinzu. Man ließ auf Raumtemperatur (= 25°C) erwärmen, gab die Mischung auf ca. 3 l Ammoniumchlorid-Lösung und extrahierte dreimal mit Methyl-tert.-Butylether. Die vereinigten organischen Phasen wurden über Na₂SO₄ getrocknet und einrotiert. Man erhielt 200 g Rohprodukt, aus dem man 118 g der Zielverbindung mittels Kieselgelchromatographie (Laufmittel: Toluol) isolierte.
   ¹H-NMR (CDCl₃, δ in ppm):
   2,6 (s, 3H); 3,9 (s, 3H); 7,25 (dd, 1H); 7,55 (d, 1H); 7,6 (d, 1H)
b) α-(3-Chlor-2-methylphenyl)-β-methoxyacrylsäuremethylester
   92,3 g Methoxymethyl-triphenylphosphonium-chlorid wurden in 500 ml wasserfreiem Dimethylformamid suspendiert und mit 42,3 g 30 %iger Natriummethanolat-Lösung versetzt. Nach 20 min tropfte man zu der hellgelben Suspension eine Lösung von 30 g 3-Chlor-2-methylphenyl-glyoxylsäuremethylester in 200 ml Dimethylformamid. Nach weiteren 3 Stunden bei Raumtemperatur gab man auf Eiswasser und extrahierte 3x mit Methyl-tert.butylether. Man trocknete über Natriumsulfat, engte ein und zog das Rohprodukt auf 100 g Kieselgel auf. Mit Methyl-tert.butylether wurde das Produkt über eine Kieselsäule eluiert. Man erhielt 29 g eines farblosen Feststoffs.
   Fp. [°C]: 66-68
   ¹H-NMR (CDCl₃, δ in ppm):
   2,2 (s, 3H); 3,7 (s, 3H); 3,85 (s, 3H); 7,05 (d, 1H); 7,15 (dd, 1H); 7,35 (d, 1H); 7,55 (s, 1H)
c) α-(2-Brommethyl-3-chlorphenyl)-β-methoxyacrylsäuremethylester
   27,0 g α-(3-Chlor-2-methylphenyl)-b-methoxyacrylsäuremethylester und 21,5 g N-Bromsuccinimid wurden in 300 ml wasserfreiem Tetrachlormethan gerührt. Nach Zugabe von 1 g Porofor N wurde 4 h auf Rückfluß erhitzt. Nach Abkühlen auf Raumtemperatur (= 25°C) wurde das Succinimid entfernt. Die vereinigten organischen Phasen wurden mit Wasser gewaschen, über Natriumsulfat getrocknet und eingeengt. Man erhielt 23 g der Titelverbindung als farblose Kristalle.
   Fp. [°C]: 66-68
   ¹H-NMR (CDCl₃, δ in ppm):
   3,75 (s, 3H); 3,85 (s, 3H); 4,5 (breit, 2H); 7,05 (d, 1H); 7,2 (dd, 1H); 7,4 (d, 1H); 7,65 (s, 1H)
d) α-(3-Chlor-2-[-(1-[2,4-dichlorphenyl]-pyrazol-3-yl)-oxymethyl]-phenyl-b-methoxyacrylsäuremethylester
   1,6 g 1-(2,4-Dichlor-phenyl)-3-hydroxy-pyrazol und 0,97 g Kaliumcarbonat wurden in 20 ml Dimethylformamid ca. 30 min gerührt. Dann gab man eine Lösung von 2,23 g a-(2-Brommethyl-3-chlor-phenyl)-b-methoxyacrylsäuremethylester in 20 ml Dimethylformamid hinzu. Nach einigen Stunden bei Raumtemperatur erwärmte man den Ansatz für 4 h auf 60°C. Zur Aufarbeitung wurde auf Eiswasser und 3x mit Methyl-tert.butylether extrahiert. Die vereinigten organischen Phasen wurden über Natriumsulfat getrocknet eingeengt und das Rohprodukt wurde über Kieselgel chromatographiert (Laufmittel Toluol). Man erhielt 1,7 g der Titelverbindung als zähes Öl.
   1H-NMR (CDCl3, □ in ppm):
   3,7 (s, 3H); 3,8 (s, 3H); 5,35 (s, 2H); 5,9 (d, 1H); 7,1-7,5 (m, 5H); 7,55 (s, 1H); 7,6 (d, 1H); 7,7 (d, 1H)

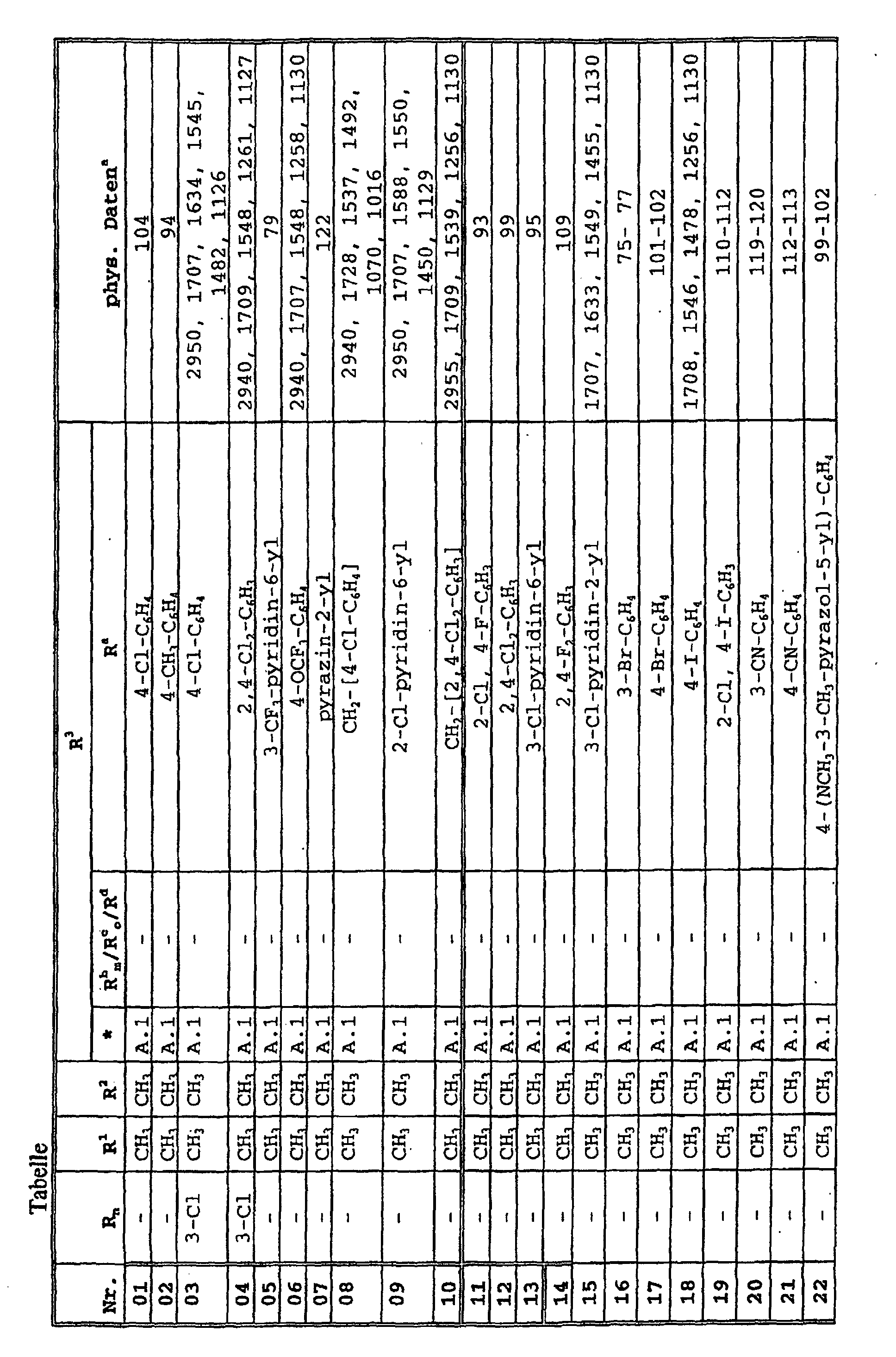

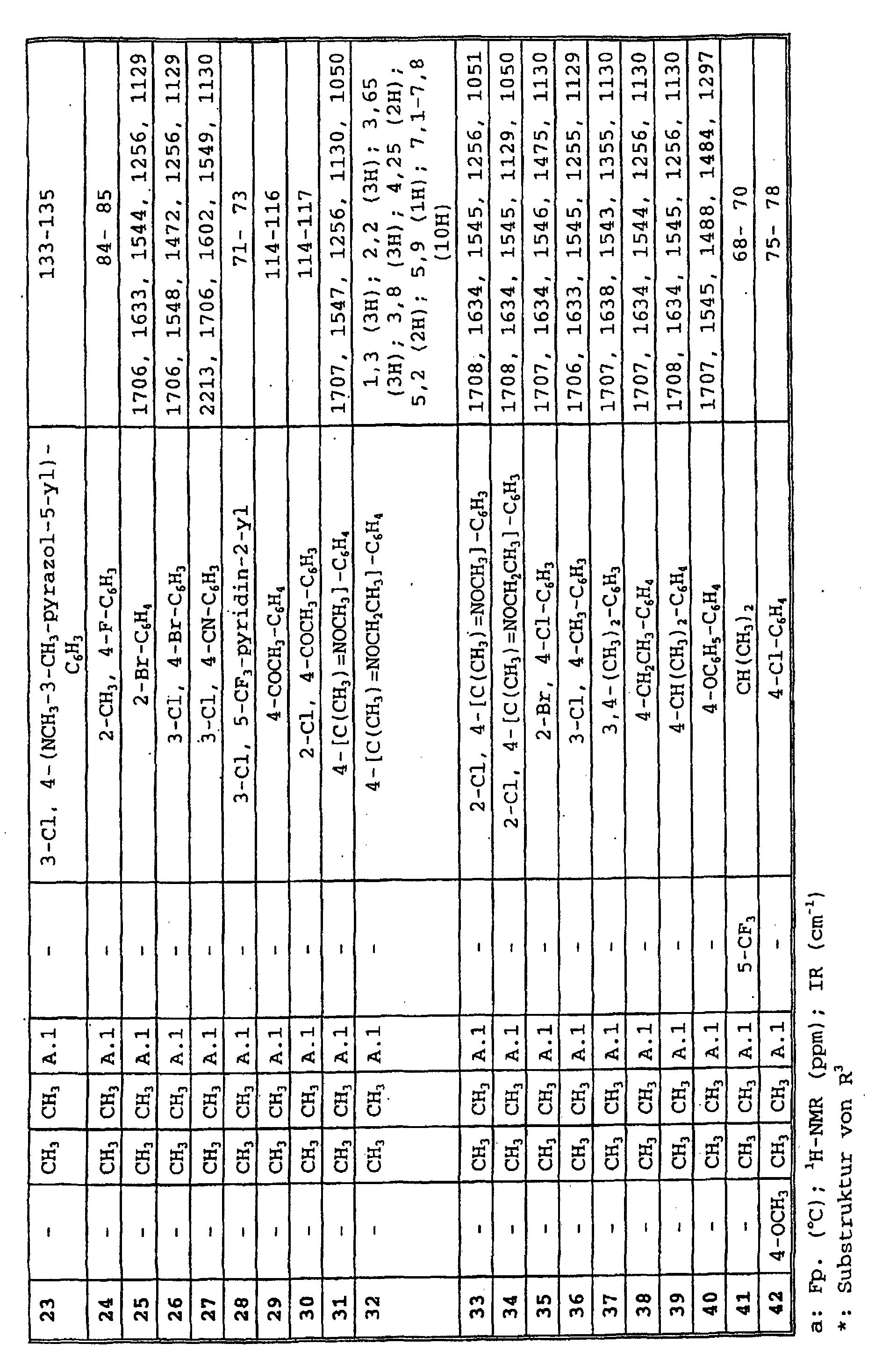

### Beispiele zur Wirkung gegen Schadpilze

Die fungizide Wirkung der Verbindungen der Formel I ließ sich durch folgende Versuche zeigen:

Die Wirkstoffe wurden als 20 %-ige Emulsion in einem Gemisch aus 70 Gew.-% Cyclohexanon, 20 Gew.-% NekanilR LN (LutensolR AP6, Netzmittel mit Emulgier- und Dispergierwirkung auf der Basis ethoxylierter Alkylphenole) und 10 Gew.-% EmulphorR EL (EmulanR EL, Emulgator auf der Basis ethoxylierter Fettalkohole) aufbereitet und entsprechend der gewünschten Konzentration mit Wasser verdünnt.

### Wirkung gegen Plasmopara viticola (Rebenperonospora)

Topfreben (Sorte: "Müller Thurgau") wurden mit der Wirkstoffaufbereitung tropfnaß gespritzt (Aufwandmenge: 16 ppm). Nach 8 Tagen wurden die Pflanzen mit einer Zoosporenaufschwemmung des Pilzes *Plasmopara viticola* besprüht und 5 Tage bei 20-30°C bei hoher Luftfeuchtigkeit bewahrt. Vor der Beurteilung wurden die Pflanzen danach für 16 h bei hoher Luftfeuchtigkeit bewahrt. Die Auswertung erfolgte visuell.

In diesem Test zeigten die mit den erfindungsgemäßen Verbindungen Nrs. 1-6, 8, 10 und 11-14 behandelten Pflanzen einen Befall von 15% und weniger während die unbehandelten (Kontroll-) Pflanzen zu 70% befallen waren.

In einem entsprechenden Test zeigten die mit den erfindungsgemäßen Verbindungen 15-22, 24-26 und 28-41 behandelten Pflanzen einen Befall von 15% und weniger während die unbehandelten (Kontroll-) Pflanzen zu 70% befallen waren.

### Wirkung gegen Puccinia recondita (Weizenbraunrost)

Blätter von Weizensämlingen (Sorte "Kanzler") wurden mit Sporen des Braunrosts (*Puccinia recondita*) bestäubt. Die so behandelten Pflanzen wurden 24 h bei 20-22°C und einer relativen Luftfeuchtigkeit von 90-95% inkubiert und anschließend mit der wäßrigen Wirkstoffaufbereitung behandelt (Aufwandmenge: 63 ppm). Nach weiteren 8 Tagen bei 20-22°C und 65-70% relativer Luftfeuchtigkeit wurde das Ausmaß der Pilzentwicklung ermittlelt. Die Auswertung erfolgte visuell.

In diesem Test zeigten die mit den erfindungsgemäßen Verbindungen Nrs. 1-6 und 9-14 behandelten Pflanzen einen Befall von 15% und weniger während die unbehandelten (Kontroll-) Pflanzen zu 65% befallen waren.

In einem entsprechenden Test zeigten die mit den erfindungsgemäßen Verbindungen 15-22, 24-26, 28, 31-33, 35-40 und 42 behandelten Pflanzen einen Befall von 15% und weniger während die unbehandelten (Kontroll-) Pflanzen zu 65% befallen waren.

### Wirkung gegen Pyricularia oryzae (Reisbrand)

Reiskeimlinge (Sorte: "Tai Nong 67") wurden mit der Wirkstoffaufbereitung tropfnaß gespritzt (Aufwandmenge: 63 ppm). Nach 24 Stunden wurden die Pflanzen mit einer wäßrigen Sporensuspension des Pilzes *Pyricularia oryzae* besprüht und 6 Tage bei 22-24°C bei einer relativen Luftfeuchtigkeit von 95-99% bewahrt. Die Beurteilung erfolgte visuell.

In diesem Test zeigten die mit den erfindungsgemäßen Verbindungen Nrs. 1-6, 9, 10, 12 und 14 behandelten Pflanzen einen Befall von 15% und weniger während die unbehandelten (Kontroll-) Pflanzen zu 85% befallen waren.

In einem entsprechenden Test zeigten die mit den erfindungsgemäßen Verbindungen 15, 18-21, 25-28, 32, 33 und 35-41 behandelten Pflanzen einen Befall von 15% und weniger während die unbehandelten (Kontroll-) Pflanzen zu 65% befallen waren.

### Beispiele zur Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% NekanilR LN (LutensolR AP6, Netzmittel mit Emulgier- und Dispergierwirkung auf der Basis ethoxylierter Alkylphenole) und 10 Gew.-% EmulphorR EL (EmulanR EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

### Aphis fabae (Schwarze Laus), Kontaktwirkung

Stark befallene Buschbohnen (Vicia faba) wurden mit der wässrigen Wirkstoffaufbereitung behandelt. Nach 24h wurde die Mortalitätsrate bestimmt.

In diesem Test zeigten die Verbindungen 17, 37 und 39-41 Wirkschwellen von 400 ppm und weniger.

### Nephotettix cincticeps (Grüne Reiszikade), Kontaktwirkung

Rundfilter wurden mit der wäßrigen Wirkstoffaufbereitung behandelt und anschließend mit 5 adulten Zikaden belegt. Nach 24h wurde die Mortalität beurteilt.

In diesem Test zeigten die Verbindungen 15, 26, und 42 Wirkschwellen von 0,4 mg und weniger.

### Prodenia litura (Ägyptischer Baumwollwurm), Kontaktwirkung

Mit der wäßrigen Wirkstoffaufbereitung behandelte Filter wurden mit 5 Raupen belegt. Die erste Beurteilung erfolgt nach 4h. Sofern noch mindestens eine Raupe lebt, wird eine Futtermischung zugegeben. Nach 24h wurde die Mortalität bestimmt.

In diesem Test zeigten die Verbindungen 18 und 26 Wirkschwellen von 0,4 mg und weniger.

### Tetranychus telarius (Rote Spinne), Kontaktwirkung

Stark befallene getopfte Buschbohnen, die das zweite Folgeblattpaar zeigten, wurden mit wässrigen Wirkstoffaufbereitung behandelt. Nach 5 Tagen im Gewächshaus wurde der Bekämpfungserfolg mittels Binokular bestimmt.

In diesem Test zeigten die Verbindungen 18, 19 und 35-42 Wirkschwellen von 400 ppm und weniger.

## Patentansprüche

1. Verbindungen der allgemeinen Formel I 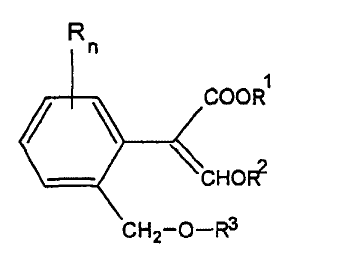 in der der Index und die Substituenten die folgende Bedeutung haben:
n 0, 1, 2, 3 oder 4, wobei die Substituenten R verschieden sein können, wenn n größer als 1 ist;
R Nitro, Cyano, Halogen,
C₁-C₁₀-Alkyl, C₂-C₁₀-Alkenyl, C₂-C₁₀-Alkinyl, C₁-C₁₀-Alkoxy, C₂-C₁₀-Alkenyloxy, C₂-C₁₀-Alkinyloxy oder
für den Fall, daß n größer als 1 ist, zusätzlich eine an zwei benachbarte Ringatome gebundene Brücke, welche drei bis vier Glieder aus der Gruppe 3 oder 4 Kohlenstoffatome 1, 2 oder 3 Kohlenstoffatome und 1 oder 2 Stickstoff-, Sauerstoff- und/oder Schwefelatome, wobei diese Brücke gemeinsam mit dem Ring an den sie gebunden ist einen partiell ungesättigten oder aromatischen Rest bilden kann;
wobei die aliphatischen Gruppen partiell oder vollständig halogeniert sein können und/oder einen bis drei, insbesondere einen der folgenden Reste tragen können:
Nitro, Cyano, C₁-C₄-Alkoxy, C₁-C₄-Alkoxycarbonyl oder eine Gruppe = N-OR^{x}, in der R^{x} für C₁-C₆-Alkyl, C₃-C₆-Alkenyl, C₃-C₆-Alkinyl steht,
oder
oder ein ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff-oder Schwefelatom als Ringglieder enthalten kann, welchees wiederum partiell oder vollständig halogeniert sein kann und/oder einen bis drei der folgenden Reste tragen kann: Nitro, Cyano, C₁-C₄-Alkyl, C₁-C₄-Alkoxy, C₁-C₄-Alkoxycarbonyl;
R¹, R² C₁-C₄-Alkyl;
R³ ein Pyrazolrest der Formeln A 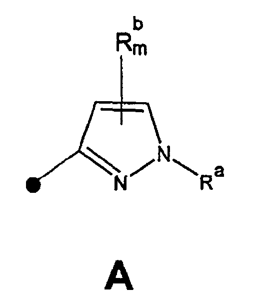 wobei die mit • gekennzeichnete Bindung der Bindung zum Sauerstoff entspricht und in denen der Index und die Substituenten die folgende Bedeutung haben:
R^{a} ein partiell oder vollständig halogenierter und/oder durch einen bis drei der folgenden Reste: Nitro, Cyano, C₁-C₄-Alkyl, C₁-C₄-Alkoxy, C₁-C₄-Alkoxycarbonyl, substituierter ein- oder zweikerniger aromatischer Rest, welcher neben Kohlenstoffatomen ein bis vier Stickstoffatome oder ein oder zwei Stickstoffatome und ein oder zwei Sauerstoff-oder Schwefelatome oder ein Sauerstoff- oder ein Schwefelatom als Ringglieder enthalten kann;
m 0, 1 oder 2, wobei die Substituenten R^{b} verschieden sein können, wenn m größer als 1 ist;
R^{b} Cyano, Nitro, Halogen, C₁-C₄-Alkyl, C₁-C₄-Halogenalkyl, C₁-C₄-Alkoxy, C₁-C₄-Halogenalkoxy, C₁-C₄-Alkylthio und C₁-C₄-Alkoxycarbonyl.

2. Verbindungen der Formel I gemäß Anspruch 1 in der für R^{a} für substituiertes Aryl, insbesondere Phenyl steht.

3. Verfahren zur Herstellung der Verbindungen der Formel I, **dadurch gekennzeichnet, daß** man ein Benzylderivat der Formel II 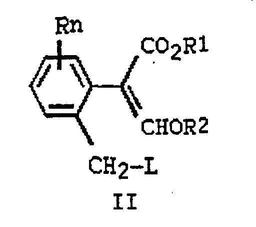 in der L eine nucleophil austauschbare Gruppe bedeutet, in Gegenwart einer Base mit einem Hydroxyazol der Formel III
HO-R³ III
umsetzt.

4. Zur Bekämpfung von Schadpilzen geeignete Mischung, enthaltend einen inerten Zusatzstoff und eine wirksame Menge einer Verbindung der Formel I gemäß Anspruch 1.

5. Zur Bekämpfung von tierischen Schädlingen geeignete Mischung, enthaltend einen inerten Zusatzstoff und eine wirksame Menge einer Verbindung der Formel I gemäß Anspruch 1.

6. Verfahren zur Bekämpfung von Schadpilzen, **dadurch gekennzeichnet, daß** man die Schadpilze, ihren Lebensraum oder die von Schadpilzen freizuhaltenden Pflanzen, Flächen, Materialien oder Räume mit einer wirksamen Menge einer Verbindung der Formel I gemäß Anspruch 1 behandelt.

7. Verfahren zur Bekämpfung von tierischen Schädlingen, **dadurch gekennzeichnet, daß** man die Schädlingen, ihren Lebensraum oder die von Schädlingen freizuhaltenden Pflanzen, Flächen, Materialien oder Räume mit einer wirksamen Menge einer Verbindung der Formel I gemäß Anspruch 1 behandelt.

8. Verwendung der Verbindungen I gemäß Anspruch 1 zur Herstellung einer zur Bekämpfung von Schadpilzen geeigneten Mischung.

9. Verwendung der Verbindungen I gemäß Anspruch 1 zur Bekämpfung von Schadpilzen.

10. Verwendung der Verbindungen I gemäß Anspruch 1 zur Bekämpfung von tierischen Schädlingen.

11. Verbindungen der Formel IV 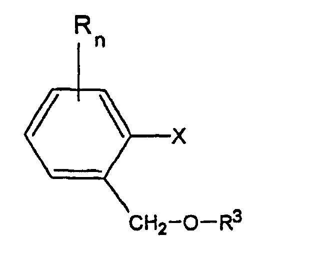 in der die Reste R und R³ sowie der Index n die in Anspruch 1 gegebene Bedeutung haben und X für CH₂CN, CH₂CO₂R¹ und C(=O)CO₂R¹ steht, wobei R¹ die in Anspruch 1 gegebene Bedeutung hat.

## Claims

1. A compound of the general formula I 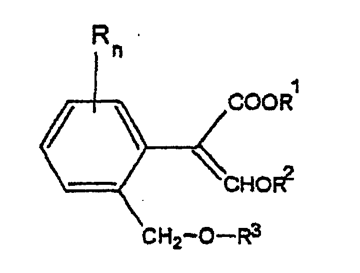 in which the index and the substituents have the following meanings:
n is 0, 1, 2, 3 or 4, it being possible for the substituents R to differ if n is greater than 1;
R is nitro, cyano, halogen,
C₁-C₁₀-alkyl, C₂-C₁₀-alkenyl, C₂-C₁₀-alkynyl,
C₁-C₁₀-alkoxy, C₂-C₁₀-alkenyloxy,
C₂-C₁₀-alkynyloxy or
in the event that n is greater than 1 additionally a bridge which is bonded to two adjacent ring atoms and which contains three to four members selected from the group consisting of 3 or 4 carbon atoms, 1, 2 or 3 carbon atoms and 1 or 2 nitrogen, oxygen and/or sulfur atoms, it being possible for this bridge, together with the ring to which it is bonded, to form a partially unsaturated or aromatic radical;
where the aliphatic groups can be partially or fully halogenated and/or can have attached to them one to three, in particular one, of the following radicals:
nitro, cyano, C₁-C₄-alkoxy, C₁-C₄-alkoxycarbonyl or a group =N-OR^{x}, in which R^{x} is C₁-C₆-alkyl, C₃-C₆-alkenyl, C₃-C₆-alkynyl,
or
or a mono- or binuclear aromatic ring system which, in addition to carbon atoms, may comprise one to four nitrogen atoms or one or two nitrogen atoms and one oxygen or sulfur atom or one oxygen or sulfur atom as ring members and which, in turn, can be partially or fully halogenated and/or can have attached to it one to three of the following radicals: nitro, cyano, C₁-C₄-alkyl, C₁-C₄-alkoxy, C₁-C₄-alkoxycarbonyl;
R¹,R² are C₁-C₄-alkyl;
R³ is a pyrazolyl radical of the formulae A 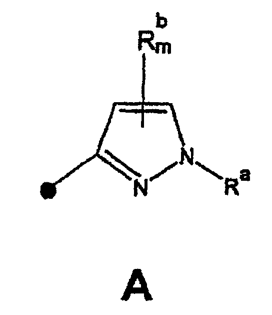 the bond marked with • being the bond to the oxygen and the index and the substituents having the following meanings:
R^{a} is a mono- or binuclear aromatic radical which is partially or fully halogentaed and/or is substituted by one to three of the following radicals: nitro, cyano, C₁-C₄-alkyl, C₁-C₄-alkoxy, C₁-C₄-alkoxycarbonyl, and which, besides carbon atoms, can contain one to four nitrogen atoms or one or two nitrogen atoms and one or two oxygen or sulfur atoms or one oxygen or one sulfur atom as ring members;
m is 0, 1 or 2, it being possible for the substituents R^{b} to differ if m is greater than 1;
R^{b} is cyano, nitro, halogen, C₁-C₄-alkyl, C₁-C₄-haloalkyl, C₁-C₄-alkoxy, C₁-C₄-haloalkoxy, C₁-C₄-alkylthio and C₁-C₄-alkoxycarbonyl;

2. A compound of the formula I as claimed in claim 1, in which for [sic] R^{a} is substituted aryl, in particular phenyl.

3. A process for the preparation of the compounds of the formula I , which comprises reacting a benzyl derivative of the formula II 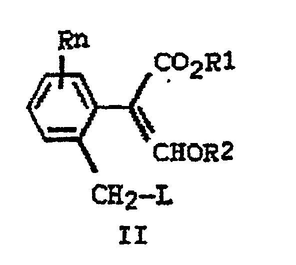 where L is a nucleophilically exchangeable group with a hydroxyazole of the formula III
HO-R³ III
in the presence of a base.

4. A mixture suitable for controlling fungal pests, comprising an inert additive and an effective amount of a compound of the formula I as claimed in claim 1.

5. A mixture which is suitable for controlling animal pests, comprising an inert additive and an effective amount of a compound of the formula I as claimed in claim 1.

6. A method of controlling fungal pests, which comprises treating the fungal pests, their environment or the plants, areas, materials or rooms to be protected against fungal pests with an effective amount of a compound of the formula I as claimed in claim 1.

7. A method of controlling animal pests, which comprises treating the pests, their environment or the plants, areas, materials or rooms to be protected against pests with an effective amount of a compound of the formula I as claimed in claim 1.

8. The use of the compounds I as claimed in claim 1 for the preparation of a mixture which is suitable for controlling fungal pests.

9. The use of the compounds I as claimed in claim 1 for controlling fungal pests.

10. The use of the compounds I as claimed in claim 1 for controlling animal pests.

11. A compound of the formula IV 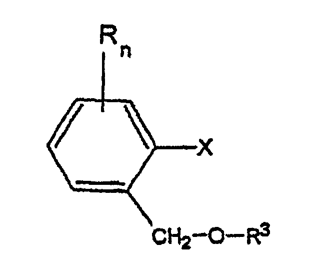 where the radicals R and R³ and the index n have the meanings given in claim 1 and X is CH₂CN, CH₂CO₂R¹ and C(=O)CO₂R¹ where R¹ has the meanings given in claim 1.

## Revendications

1. Composés de formule générale I 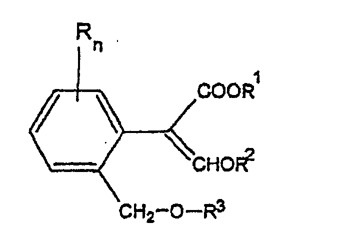 dans laquelle l'indice et les substituants présentent la signification suivante :
n vaut 0, 1, 2, 3 ou 4, les substituants R pouvant être différents si n est supérieur à 1 ;
R représente un nitro, un cyano, un halogène,
un alkyle en C₁-C₁₀, un alcényle en C₂-C₁₀, un alcynyle en C₂-C₁₀, un alcoxy en C₁-C₁₀, un alcényloxy en C₂-C₁₀, un alcynyloxy en C₂-C₁₀ ou
au cas où n est supérieur à 1, en outre un pont lié à deux atomes cycliques voisins, lequel renferme trois à quatre chaînons parmi le groupe constitué de 3 ou 4 atomes de carbone, de 1, 2 ou 3 atomes de carbone et de 1 ou 2 atomes d'azote, d'oxygène et/ou de soufre, ce pont pouvant former, conjointement avec le noyau auquel il est lié, un radical partiellement insaturé ou aromatique ;
les groupes aliphatiques pouvant être partiellement ou totalement halogénés et/ou pouvant porter un à trois, en particulier un des radicaux suivants :
un nitro, un cyano, un alcoxy en C₁-C₄, un C₁-C₄-alcoxycarbonyle ou un groupe = N-OR^{x}, dans lequel R^{x} représente un alkyle en C₁-C₆, un alcényle en C₃-C₆ ou un alcynyle en C₃-C₆,
ou
un système cyclique aromatique mono- ou binucléaire qui peut renfermer, en plus d'atomes de carbone, un à quatre atomes d'azote ou un ou deux atomes d'azote et un atome d'oxygène ou de soufre ou un atome d'oxygène ou de soufre en tant que chaînons cycliques, lequel peut être de nouveau partiellement ou totalement halogéné et/ou peut porter un à trois des radicaux suivants : un nitro, un cyano, un alkyle en C₁-C₄, un alcoxy en C₁-C₄, un C₁-C₄-alcoxycarbonyle ;
R¹, R² représentent un alkyle en C₁-C₄ ;
R³ représente un radical pyrazole de formule A 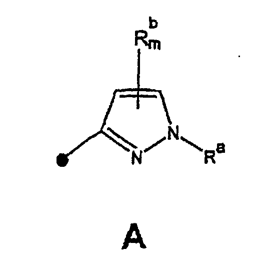 dans laquelle la liaison marquée par correspond à la liaison à l'oxygène et l'indice et les substituants présentent la signification suivante :
R^{a} représente un radical aromatique mono- ou binucléaire, partiellement ou totalement halogéné et/ou substitué par un à trois des radicaux suivants : un nitro, un cyano, un alkyle en C₁-C₄, un alcoxy en C₁-C₄, un C₁-C₄-alcoxycarbonyle, lequel peut renfermer, en plus d'atomes de carbone, un à quatre atomes d'azote ou un ou deux atomes d'azote et un ou deux atomes d'oxygène ou de soufre ou un atome d'oxygène ou de soufre en tant que chaînons cycliques ;
m vaut 0, 1 ou 2, les substituants R^{b} pouvant être différents si m est supérieur à 1 ;
R^{b} représente un cyano, un nitro, un halogène, un alkyle en C₁-C₄, un halogénoalkyle en C₁-C₄, un alcoxy en C₁-C₄, un halogénoalcoxy en C₁-C₄, un alkylthio en C₁-C₄ et un C₁-C₄-alcoxycarbonyle.

2. Composés de formule I selon la revendication 1, dans laquelle R^{a} représente un aryle substitué, en particulier un phényle.

3. Procédé de préparation des composés de formule I, **caractérisé en ce qu'**un dérivé benzylique de formule II 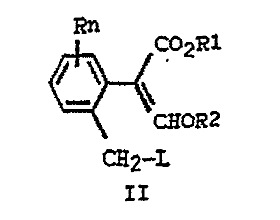 dans laquelle L représente un groupe remplaçable par voie nucléophile, est mis à réagir en présence d'une base, avec un hydroxyazole de formule III
HO-R³ III .

4. Mélange approprié pour la lutte contre des champignons nuisibles, comprenant un additif inerte et une quantité efficace d'un composé de formule I selon la revendication 1.

5. Mélange approprié pour la lutte contre des parasites animaux, comprenant un additif inerte et une quantité efficace d'un composé de formule I selon la revendication 1.

6. Procédé de lutte contre les champignons nuisibles,
**caractérisé en ce que** les champignons nuisibles, leur environnement ou les plantes, les surfaces, les matériaux ou les pièces à protéger vis-à-vis de champignons nuisibles sont traités par une quantité efficace d'un composé de formule I selon la revendication 1.

7. Procédé de lutte contre les parasites animaux, **caractérisé en ce que** les parasites animaux, leur environnement ou les plantes, les surfaces, les matériaux ou les pièces à protéger vis-à-vis de parasites sont traités par une quantité efficace d'un composé de formule I selon la revendication 1.

8. Utilisation des composés I selon la revendication 1 pour la préparation d'un mélange approprié pour la lutte contre des champignons nuisibles.

9. Utilisation des composés I selon la revendication 1 pour la lutte contre des champignons nuisibles.

10. Utilisation des composés I selon la revendication 1 pour la lutte contre des parasites animaux.

11. Composés de formule IV 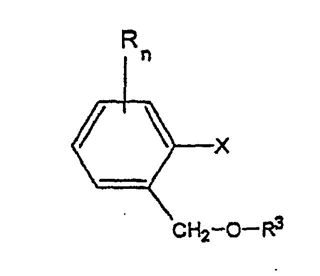 dans laquelle les radicaux R et R³ ainsi que l'indice n présentent la signification indiquée dans la revendication 1 et X représente CH₂CN, CH₂CO₂R¹ et C(=O)CO₂R¹, R¹ présentant la signification indiquée dans la revendication 1.
